# EUROPEAN PATENT SPECIFICATION

(11) **EP 4 006 171 B1**
(45) Date of publication and mention of the grant of the patent: **10.12.2025**
(21) Application number: 20846161.6
(22) Date of filing: 28.07.2020
(51) Int. Cl.: C12Q 1/6886, C12N 15/11

(54) **TUMOUR MARKER AND USE THEREOF**
TUMORMARKER UND VERWENDUNG DAVON
MARQUEUR TUMORAL ET SON UTILISATION

(30) Priority: 30.07.2019 CN 201910695955
(43) Date of publication of application: 01.06.2022
(73) Proprietor: Lisen Imprinting Diagnostics Wuxi Co., Ltd, Wuxi City, Jiangsu 214000 (CN)
(72) Inventor: CHENG, Tong, Wuxi, Jiangsu 214000 (CN); ZHOU, Ning, Wuxi, Jiangsu 214000 (CN)
(74) Representative: Grund, Martin
(86) International application number: PCT/CN2020/105047
(87) International publication number: WO 2021/018116

(56) References cited:
- WO-A1-2007/063118
- WO-A1-2007/097741
- WO-A1-2007/097741
- WO-A1-2014/071029
- WO-A1-2017/158158
- WO-A1-95/24503
- WO-A2-2006/137066
- WO-A2-2008/079269
- CN-A- 105 154 542
- CN-A- 105 408 494
- CN-A- 107 723 368
- CN-A- 108 368 552
- CN-A- 108 977 535
- JP-A- 2012 090 555
- US-A1- 2012 004 127

## Description

### Technical Field

This application pertains to the field of biotechnology, in particular the field of genetic diagnosis technology, and relates to a tumor marker and its applications. More particularly, the application relates to the use of an imprinted gene-based tumor marker, of a tumor diagnosis model based on the imprinted gene-based tumor marker, or of a tumor diagnosis device based on the imprinted gene-based tumor marker in preparing a drug and/or a reagent for use in diagnosing, screening, or treating a tumor.

### Description of Related Art

Malignant tumors are one of the major factors that pose a serious threat to human health. Each year there are 14 million new cases of cancer patients and 8.2 million deaths caused by cancer globally. Infinite proliferation and metastasis are the two major features of cancer cells, and most cancer patients die because the cancer cells metastasize rapidly and cause such complications as organ failure. Early cancer lesions are relatively small in size; therefore, not only do they result in relatively small surgical wounds when treated surgically, but also only a relatively small number of cancer cells may enter the circulatory system, meaning the circulating cancer cells are relatively unlikely to form stable metastases. It follows that early/timely effective treatment of cancer is crucial to relapse prevention and life extension. Currently, the criteria of cancer diagnosis consist in cell and tissue morphology. Morphological observation, however, depends heavily on a pathologist's experience, and missed diagnosis may occur due to the fact that an early-stage cancer may not have developed typical morphological changes. Now that molecular biological changes in epigenetics and genetics take place earlier than variations in morphology during cancer development, molecular biology-based detection is more sensitive than morphology-based detection when it comes to detecting an early-stage cancer. Molecular markers are hence important to early diagnosis of cancer.

Genomic imprinting is a gene regulation method in epigenetics and is characterized by methylating an allele from a specific parent such that only one allele of the corresponding gene is expressed while the other allele is in a silenced state. A gene regulated in this way is referred to as an imprinted gene. Loss of imprinting is an epigenetic change in which the silenced allele of an imprinted gene is demethylated and is thus activated and expressed. Numerous studies have shown that loss of imprinting exists widely in all kinds of cancers and occurs earlier than morphological changes in cells and tissue. Loss of imprinting, however, seldom occurs in healthy cells, which is in stark contrast to the case with cancer cells. Therefore, the methylated state of an imprinted gene can serve as a pathological marker and used in conjunction with specific molecular detection techniques to analyze cellular abnormality.

As the functions of an imprinted gene cover cell signaling, cell cycle regulation, substance transport across the cell membrane, the formation of extracellular matrix, and so on, the expressed functions and expressed quantity of an imprinted gene in a certain cancer may differ from those in another cancer, with the difference in expressed quantity being huge. That is to say, an imprinted gene may have different sensitivity and specificity to a certain cancer from another imprinted gene. Such differences in sensitivity and specificity have a significant impact on the invasion and metastasis of tumor cells during tumor development and on the prognosis of tumors. Different combinations of imprinted genes can also be used to distinguish different types of tumors.

According to the above, highly sensitive diagnostic markers for early tumor diagnosis are currently unavailable. It is necessary to develop a new diagnostic marker, diagnostic model, and diagnostic device that can be applied to a patient's biopsy samples to analyze changes in a molecular marker on a cellular level, in order to provide more accurate prognosis and diagnosis information as well as guidance on the selection of treatment options.

WO 2007/097741 A1 discloses methods for diagnosing intrauterine growth restriction by analyzing expression levels of certain genes in samples. CN 108977535 A discloses an imprinting gene model for the detection of bladder cancer. WO 2007/063118 A1 discloses methods for classification and prognosis of hepatocellular carcinoma by analyzing expression levels of certain genes in samples. WO 95/24503 A1 discloses gene H19 as a marker of various types of human cancer.

### Brief Summary of the Invention

The invention is defined by the appended claims.

This application provides a tumor marker and its applications. An imprinted gene is used as a tumor marker for early tumor diagnosis. A tumor diagnosis model and tumor diagnosis device based on the imprinted gene-based tumor marker enable tumor diagnosis on a single-cell or tissue level and thereby provide a basis for the treatment of various malignant tumors.

The imprinted gene Z17 is Aldh1l1, the imprinted gene Z18 is Gata3, the imprinted gene Z19 is Hm13, the imprinted gene Z20 is Kcnq1, the imprinted gene Z21 is Nhp2l1, the imprinted gene Z22 is Pon2, the imprinted gene Z23 is Rasgrf1, the imprinted gene Z25 is Tfpi2, the imprinted gene Z26 is Trappc9, the imprinted gene Z27 is Ube3a, and the imprinted gene Z28 is Zic1.

In this application, the Aldh1l1 gene has the nucleotide sequence of NC_000003.12 (126103561-126197758, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Gata3 gene has the nucleotide sequence of NC_000010.11 (8045420-8075198) or has a nucleotide sequence showing at least 90%, at least 91%, at least 92%, at least 93%, at least 94%, at least 95%, at least 96%, at least 97%, at least 98%, or at least 99% homology therewith.

In this application, the Hm13 gene has the nucleotide sequence of NC_000020.11 (31514410-31569567) or has a nucleotide sequence showing at least 90%, at least 91%, at least 92%, at least 93%, at least 94%, at least 95%, at least 96%, at least 97%, at least 98%, or at least 99% homology therewith.

In this application, the Kenq1 gene has the nucleotide sequence of NC_000011.10 (2445008-2849110) or has a nucleotide sequence showing at least 90%, at least 91%, at least 92%, at least 93%, at least 94%, at least 95%, at least 96%, at least 97%, at least 98%, or at least 99% homology therewith.

In this application, the Nhp2l1 gene has the nucleotide sequence of NC_000022.11 (41673933-41690492, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Pon2 gene has the nucleotide sequence of NC_000007.14 (95404862-95435072, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Rasgrf1 gene has the nucleotide sequence of NC_000015.10 (78959906-79090780, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Tfpi2 gene has the nucleotide sequence of NC_000007.14 (93885396-93890753, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Trappc9 gene has the nucleotide sequence of NC_000008.11 (139727725-140458579, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Ube3a gene has the nucleotide sequence of NC_000015.10 (25333728-25439381, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Zic1 gene has the nucleotide sequence of NC_000003.12 (147409365-147416719) or has a nucleotide sequence showing at least 90%, at least 91%, at least 92%, at least 93%, at least 94%, at least 95%, at least 96%, at least 97%, at least 98%, or at least 99% homology therewith.

The imprinted gene Z1 is Gnas, the imprinted gene Z2 is Igf2, the imprinted gene Z3 is Peg10, the imprinted gene Z4 is Igf2r, the imprinted gene Z5 is Mest, the imprinted gene Z6 is Plagl1, the imprinted gene Z8 is Dcn, the imprinted gene Z9 is Dlk1, the imprinted gene Z10 is Gatm, the imprinted gene N11 is Grb10, the imprinted gene Z12 is Peg3, the imprinted gene Z13 is Sgce, the imprinted gene Z14 is Slc38a4, the imprinted gene Z15 is Diras3, and the imprinted gene N16 is Snrpn/Snurf.

In this application, the Gnas gene has the nucleotide sequence of NC_000020.11 (58839681-58911196) or has a nucleotide sequence showing at least 90%, at least 91%, at least 92%, at least 93%, at least 94%, at least 95%, at least 96%, at least 97%, at least 98%, or at least 99% homology therewith.

In this application, the Igf2 gene has the nucleotide sequence of NC_000011.10 (2129112-2149603, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Peg10 gene has the nucleotide sequence of NC_000007.14 (94656325-94669695) or has a nucleotide sequence showing at least 90%, at least 91%, at least 92%, at least 93%, at least 94%, at least 95%, at least 96%, at least 97%, at least 98%, or at least 99% homology therewith.

In this application, the Igf2r gene has the nucleotide sequence of NC_000006.12 (159969082-160111504) or has a nucleotide sequence showing at least 90%, at least 91%, at least 92%, at least 93%, at least 94%, at least 95%, at least 96%, at least 97%, at least 98%, or at least 99% homology therewith.

In this application, the Mest gene has the nucleotide sequence of NC_000007.14 (130486175-130506465) or has a nucleotide sequence showing at least 90%, at least 91%, at least 92%, at least 93%, at least 94%, at least 95%, at least 96%, at least 97%, at least 98%, or at least 99% homology therewith.

In this application, the Plagl1 gene has the nucleotide sequence of NC_000006.12 (143940300-144064599, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Dcn gene has the nucleotide sequence of NC_000012.12 (91140484-91182817, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Dlk1 gene has the nucleotide sequence of NC_000014.9 (100726892-100738224) or has a nucleotide sequence showing at least 90%, at least 91%, at least 92%, at least 93%, at least 94%, at least 95%, at least 96%, at least 97%, at least 98%, or at least 99% homology therewith.

In this application, the Gatm gene has the nucleotide sequence of NC_000015.10 (45361124-45402227, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Grb10 gene has the nucleotide sequence of NC_000007.14 (50590068-50793453, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Peg3 gene has the nucleotide sequence of NC_000019.10 (56810082-56840726, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Sgce gene has the nucleotide sequence of NC_000007.14 (94584980-94656205, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Slc38a4 gene has the nucleotide sequence of NC_000012.12 (46764761-46832422, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Diras3 gene has the nucleotide sequence of NC_000001.11 (68045886-68051631, complementary strands) or has a nucleotide sequence showing at least 90%, at least 91%, at least 92%, at least 93%, at least 94%, at least 95%, at least 96%, at least 97%, at least 98%, or at least 99% homology therewith.

In this application, the Snrpn/Snurf gene has the nucleotide sequence of NC_000015.10 (24823637-24978723) or has a nucleotide sequence showing at least 90%, at least 91%, at least 92%, at least 93%, at least 94%, at least 95%, at least 96%, at least 97%, at least 98%, or at least 99% homology therewith.

In a first aspect, this application provides a method of tumor diagnosis by analyzing how the aforesaid tumor markers (i.e., imprinted genes) are expressed in samples.

Expression of the aforesaid imprinted genes includes any one, or a combination of at least two, of an imprinted gene not being expressed, the imprinted gene being expressed as normal, the imprinted gene being expressed as having a loss of imprinting, and the imprinted gene being expressed as having a copy number variation.

In this application, an imprinted gene is determined as having a loss of imprinting when two red/brown marks are present in the nucleus of a cell stained with hematoxylin, and an imprinted gene is determined as having a copy number variation when more than two red/brown marks are present in the nucleus of a cell stained with hematoxylin. A copy number variation occurs when abnormal gene duplication takes place in a cancer cell such that the duplicated gene is expressed as having three or more copies of the original gene.

In more general terms, an imprinted gene is determined as not being expressed when no imprinted gene mark is present in the nucleus of a sample cell.

In more general terms, an imprinted gene is determined as being expressed as normal when only one imprinted gene mark is present in the nucleus of a sample cell.

In more general terms, an imprinted gene is determined as having a loss of imprinting when two imprinted gene marks are present in the nucleus of a sample cell.

In more general terms, an imprinted gene is determined as having a copy number variation when more than two imprinted gene marks are present in the nucleus of a sample cell.

In this application, the hematoxylin-stained marks include but are not limited to red marks and brown marks. Staining/marking in other colors is also feasible when analyzing imprinted gene expression.

According to this application, a probe/probes specific to the imprinted gene(s) to be detected in a sample is/are used for in-situ hybridization with the sample, and tumor diagnosis is carried out by analyzing whether the imprinted gene(s) in the sample cells has/have a loss of imprinting or a copy number variation.

The diagnostic method of the tumor diagnosis model is detailed as follows.

In one embodiment of this application, a bile duct tissue and/or cell sample is subjected to an analysis of the expression of the imprinted genes Z1, Z2, Z8, Z10, N11, Z14, N16, Z19, Z20, Z21, Z22, Z25, Z26, and Z27.

If the imprinted genes Z1, Z2, Z8, Z10, N11, Z14, N16, Z19, Z20, Z21, Z22, Z25, Z26, and Z27 are not expressed or are expressed as normal in the cell nuclei in the bile duct tissue and/or cell sample, the sample will be determined as a healthy bile duct tissue and/or cell sample.

If, however, the imprinted gene Z1, Z2, Z8, Z10, N11, Z14, N16, Z19, Z20, Z21, Z22, Z25, Z26, or Z27 is expressed as having a loss of imprinting and/or a copy number variation in the cell nuclei in the bile duct tissue and/or cell sample, the sample will be determined as a cholangiocarcinoma tissue and/or cell sample.

In one embodiment of this application, a brain tissue and/or cell sample is subjected to an analysis of the expression of the imprinted genes Z1, Z2, Z4, Z5, Z8, Z9, Z10, N11, Z13, Z14, N16, Z17, Z19, Z21, Z22, Z23, Z26, Z27, and Z28.

If the imprinted genes Z1, Z2, Z4, Z5, Z8, Z9, Z10, N11, Z13, Z14, N16, Z17, Z19, Z21, Z22, Z23, Z26, Z27, and Z28 are not expressed or are expressed as normal in the cell nuclei in the brain tissue and/or cell sample, the sample will be determined as a healthy brain tissue and/or cell sample.

If, however, the imprinted gene Z1, Z2, Z4, Z5, Z8, Z9, Z10, N11, Z13, Z14, N16, Z17, Z19, Z21, Z22, Z23, Z26, Z27, or Z28 is expressed as having a loss of imprinting and/or a copy number variation in the cell nuclei in the brain tissue and/or cell sample, the sample will be determined as a brain cancer tissue and/or cell sample.

In one embodiment of this application, a kidney tissue and/or cell sample is subjected to an analysis of the expression of the imprinted genes Z1, Z4, Z6, Z8, Z10, N11, Z13, N16, Z17, Z18, Z19, Z20, Z21, Z25, Z26, Z27, and Z28.

If the imprinted genes Z1, Z4, Z6, Z8, Z10, N11, Z13, N16, Z17, Z18, Z19, Z20, Z21, Z25, Z26, Z27, and Z28 are not expressed or are expressed as normal in the cell nuclei in the kidney tissue and/or cell sample, the sample will be determined as a healthy kidney tissue and/or cell sample.

If, however, the imprinted gene Z1, Z4, Z6, Z8, Z10, N11, Z13, N16, Z17, Z18, Z19, Z20, Z21, Z25, Z26, Z27, or Z28 is expressed as having a loss of imprinting and/or a copy number variation in the cell nuclei in the kidney tissue and/or cell sample, the sample will be determined as a kidney cancer tissue and/or cell sample.

In one embodiment of this application, a bone tissue and/or cell sample is subjected to an analysis of the expression of the imprinted genes Z1, Z2, Z3, Z6, Z8, Z9, Z10, N11, Z13, N16, Z18, Z19, Z20, Z21, Z22, Z25, Z26, Z27, and Z28.

If the imprinted genes Z1, Z2, Z3, Z6, Z8, Z9, Z10, N11, Z13, N16, Z18, Z19, Z20, Z21, Z22, Z25, Z26, Z27, and Z28 are not expressed or are expressed as normal in the cell nuclei in the bone tissue and/or cell sample, the sample will be determined as a healthy bone tissue and/or cell sample.

If, however, the imprinted gene Z1, Z2, Z3, Z6, Z8, Z9, Z10, N11, Z13, N16, Z18, Z19, Z20, Z21, Z22, Z25, Z26, Z27, or Z28 is expressed as having a loss of imprinting and/or a copy number variation in the cell nuclei in the bone tissue and/or cell sample, the sample will be determined as a bone cancer tissue and/or cell sample.

In one embodiment of this application, a cervical tissue and/or cell sample is subjected to an analysis of the expression of the imprinted genes Z1, Z3, Z5, Z8, Z9, Z10, N11, N16, Z17, Z19, Z20, Z21, Z22, Z23, Z26, Z27, and Z28.

If the imprinted genes Z1, Z3, Z5, Z8, Z9, Z10, N11, N16, Z17, Z19, Z20, Z21, Z22, Z23, Z26, Z27, and Z28 are not expressed or are expressed as normal in the cell nuclei in the cervical tissue and/or cell sample, the sample will be determined as a healthy cervical tissue and/or cell sample.

If, however, the imprinted gene Z1, Z3, Z5, Z8, Z9, Z10, N11, N16, Z17, Z19, Z20, Z21, Z22, Z23, Z26, Z27, or Z28 is expressed as having a loss of imprinting and/or a copy number variation in the cell nuclei in the cervical tissue and/or cell sample, the sample will be determined as a cervical cancer tissue and/or cell sample.

In one embodiment of this application, a nasopharyngeal tissue and/or cell sample is subjected to an analysis of the expression of the imprinted genes Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, and Z28.

If the imprinted genes Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, and Z28 are not expressed or are expressed as normal in the cell nuclei in the nasopharyngeal tissue and/or cell sample, the sample will be determined as a healthy nasopharyngeal tissue and/or cell sample.

If, however, the imprinted gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, or Z28 is expressed as having a loss of imprinting and/or a copy number variation in the cell nuclei in the nasopharyngeal tissue and/or cell sample, the sample will be determined as a nasopharyngeal carcinoma tissue and/or cell sample.

In one embodiment of this application, a laryngeal tissue and/or cell sample is subjected to an analysis of the expression of the imprinted genes Z1, Z3, Z6, Z8, N11, Z15, N16, Z17, Z18, Z19, Z21, Z22, Z25, Z26, Z27, and Z28.

If the imprinted genes Z1, Z3, Z6, Z8, N11, Z15, N16, Z17, Z18, Z19, Z21, Z22, Z25, Z26, Z27, and Z28 are not expressed or are expressed as normal in the cell nuclei in the laryngeal tissue and/or cell sample, the sample will be determined as a healthy laryngeal tissue and/or cell sample.

If, however, the imprinted gene Z1, Z3, Z6, Z8, N11, Z15, N16, Z17, Z18, Z19, Z21, Z22, Z25, Z26, Z27, or Z28 is expressed as having a loss of imprinting and/or a copy number variation in the cell nuclei in the laryngeal tissue and/or cell sample, the sample will be determined as a laryngeal cancer tissue and/or cell sample.

In one embodiment of this application, a prostate tissue and/or cell sample is subjected to an analysis of the expression of the imprinted genes Z18, Z19, Z20, Z21, Z25, Z26, Z27, and Z28.

If the imprinted genes Z18, Z19, Z20, Z21, Z25, Z26, Z27, and Z28 are not expressed or are expressed as normal in the cell nuclei in the prostate tissue and/or cell sample, the sample will be determined as a healthy prostate tissue and/or cell sample.

If, however, the imprinted gene Z18, Z19, Z20, Z21, Z25, Z26, Z27, or Z28 is expressed as having a loss of imprinting and/or a copy number variation in the cell nuclei in the prostate tissue and/or cell sample, the sample will be determined as a prostate cancer tissue and/or cell sample.

In one embodiment of this application, a gastric tissue and/or cell sample is subjected to an analysis of the expression of the imprinted genes Z18, Z19, Z20, Z21, Z22, Z26, and Z27.

If the imprinted genes Z18, Z19, Z20, Z21, Z22, Z26, and Z27 are not expressed or are expressed as normal in the cell nuclei in the gastric tissue and/or cell sample, the sample will be determined as a healthy gastric tissue and/or cell sample.

If, however, the imprinted gene Z18, Z19, Z20, Z21, Z22, Z26, or Z27 is expressed as having a loss of imprinting and/or a copy number variation in the cell nuclei in the gastric tissue and/or cell sample, the sample will be determined as a gastric carcinoma tissue and/or cell sample.

In one embodiment of this application, an esophageal tissue and/or cell sample is subjected to an analysis of the expression of the imprinted genes Z18, Z19, Z21, Z22, Z26, Z27, and Z28.

If the imprinted genes Z18, Z19, Z21, Z22, Z26, Z27, and Z28 are not expressed or are expressed as normal in the cell nuclei in the esophageal tissue and/or cell sample, the sample will be determined as a healthy esophageal tissue and/or cell sample.

If, however, the imprinted gene Z18, Z19, Z21, Z22, Z26, Z27, or Z28 is expressed as having a loss of imprinting and/or a copy number variation in the cell nuclei in the esophageal tissue and/or cell sample, the sample will be determined as an esophageal cancer tissue and/or cell sample.

In one embodiment of this application, a mammary tissue and/or cell sample is subjected to an analysis of the expression of the imprinted genes Z18, Z19, Z21, Z22, and Z27.

If the imprinted genes Z18, Z19, Z21, Z22, and Z27 are not expressed or are expressed as normal in the cell nuclei in the mammary tissue and/or cell sample, the sample will be determined as a healthy mammary tissue and/or cell sample.

If, however, the imprinted gene Z18, Z19, Z21, Z22, or Z27 is expressed as having a loss of imprinting and/or a copy number variation in the cell nuclei in the mammary tissue and/or cell sample, the sample will be determined as a mammary cancer tissue and/or cell sample.

In one embodiment of this application, a thyroid tissue and/or cell sample is subjected to an analysis of the expression of the imprinted genes Z18, Z19, Z20, Z21, and Z23.

If the imprinted genes Z18, Z19, Z20, Z21, and Z23 are not expressed or are expressed as normal in the cell nuclei in the thyroid tissue and/or cell sample, the sample will be determined as a healthy thyroid tissue and/or cell sample.

If, however, the imprinted gene Z18, Z19, Z20, Z21, or Z23 is expressed as having a loss of imprinting and/or a copy number variation in the cell nuclei in the thyroid tissue and/or cell sample, the sample will be determined as a thyroid cancer tissue and/or cell sample.

In one embodiment of this application, a bladder tissue and/or cell sample is subjected to an analysis of the expression of the imprinted genes Z18, Z19, Z20, Z21, Z22, Z26, Z27, and Z28.

If the imprinted genes Z18, Z19, Z20, Z21, Z22, Z26, Z27, and Z28 are not expressed or are expressed as normal in the cell nuclei in the bladder tissue and/or cell sample, the sample will be determined as a healthy bladder tissue and/or cell sample.

If, however, the imprinted gene Z18, Z19, Z20, Z21, Z22, Z26, Z27, or Z28 is expressed as having a loss of imprinting and/or a copy number variation in the cell nuclei in the bladder tissue and/or cell sample, the sample will be determined as a bladder cancer tissue and/or cell sample.

In one embodiment of this application, a liver tissue and/or cell sample is subjected to an analysis of the expression of the imprinted genes Z17, Z19, Z21, Z22, Z26, Z27, and Z28.

If the imprinted genes Z17, Z19, Z21, Z22, Z26, Z27, and Z28 are not expressed or are expressed as normal in the cell nuclei in the liver tissue and/or cell sample, the sample will be determined as a healthy liver tissue and/or cell sample.

If, however, the imprinted gene Z17, Z19, Z21, Z22, Z26, Z27, or Z28 is expressed as having a loss of imprinting and/or a copy number variation in the cell nuclei in the liver tissue and/or cell sample, the sample will be determined as a liver cancer tissue and/or cell sample.

In one embodiment of this application, a pancreatic tissue and/or cell sample is subjected to an analysis of the expression of the imprinted genes Z17, Z18, Z19, Z20, Z21, Z22, Z26, Z27, and Z28.

If the imprinted genes Z17, Z18, Z19, Z20, Z21, Z22, Z26, Z27, and Z28 are not expressed or are expressed as normal in the cell nuclei in the pancreatic tissue and/or cell sample, the sample will be determined as a healthy pancreatic tissue and/or cell sample.

If, however, the imprinted gene Z17, Z18, Z19, Z20, Z21, Z22, Z26, Z27, or Z28 is expressed as having a loss of imprinting and/or a copy number variation in the cell nuclei in the pancreatic tissue and/or cell sample, the sample will be determined as a pancreatic cancer tissue and/or cell sample.

In one embodiment of this application, a lymphatic tissue and/or cell sample is subjected to an analysis of the expression of the imprinted genes Z18, Z19, Z21, Z22, Z26, Z27, and Z28.

If the imprinted genes Z18, Z19, Z21, Z22, Z26, Z27, and Z28 are not expressed or are expressed as normal in the cell nuclei in the lymphatic tissue and/or cell sample, the sample will be determined as a healthy lymphatic tissue and/or cell sample.

If, however, the imprinted gene Z18, Z19, Z21, Z22, Z26, Z27, or Z28 is expressed as having a loss of imprinting and/or a copy number variation in the cell nuclei in the lymphatic tissue and/or cell sample, the sample will be determined as a lymphoma tissue and/or cell sample.

In one embodiment of this application, a colorectal tissue and/or cell sample is subjected to an analysis of the expression of the imprinted genes Z19, Z20, Z21, Z22, Z26, and Z27.

If the imprinted genes Z19, Z20, Z21, Z22, Z26, and Z27 are not expressed or are expressed as normal in the cell nuclei in the colorectal tissue and/or cell sample, the sample will be determined as a healthy colorectal tissue and/or cell sample.

If, however, the imprinted gene Z19, Z20, Z21, Z22, Z26, or Z27 is expressed as having a loss of imprinting and/or a copy number variation in the cell nuclei in the colorectal tissue and/or cell sample, the sample will be determined as a colorectal cancer tissue and/or cell sample.

In one embodiment of this application, a skin tissue and/or cell sample is subjected to an analysis of the expression of the imprinted genes Z18, Z19, Z21, Z22, Z25, and Z28.

If the imprinted genes Z18, Z19, Z21, Z22, Z25, and Z28 are not expressed or are expressed as normal in the cell nuclei in the skin tissue and/or cell sample, the sample will be determined as a healthy skin tissue and/or cell sample.

If, however, the imprinted gene Z18, Z19, Z21, Z22, Z25, or Z28 is expressed as having a loss of imprinting and/or a copy number variation in the cell nuclei in the skin tissue and/or cell sample, the sample will be determined as a skin cancer tissue and/or cell sample.

In one embodiment of this application, a lung tissue and/or cell sample is subjected to an analysis of the expression of the imprinted genes Z18, Z19, Z21, Z22, Z25, Z26, Z27, and Z28.

If the imprinted genes Z18, Z19, Z21, Z22, Z25, Z26, Z27, and Z28 are not expressed or are expressed as normal in the cell nuclei in the lung tissue and/or cell sample, the sample will be determined as a healthy lung tissue and/or cell sample.

If, however, the imprinted gene Z18, Z19, Z21, Z22, Z25, Z26, Z27, or Z28 is expressed as having a loss of imprinting and/or a copy number variation in the cell nuclei in the lung tissue and/or cell sample, the sample will be determined as a lung cancer tissue and/or cell sample.

In a second aspect, this application provides a tumor diagnosis device that includes the following units:
a sampling unit for obtaining a sample;
a probe designing unit for designing a probe specific to the sequence of an imprinted gene to be detected;
a detection unit for performing in-situ hybridization between the probe and the sample; and
an analysis unit for analyzing expression of the imprinted gene through microscopic imaging;
wherein the tumor diagnosis device is configured to carry out tumor diagnosis by analyzing how the imprinted gene(s) of a tumor marker stated in relation to the first aspect is/are expressed in the sample.

In this application, an imprinted gene is determined as having a loss of imprinting when two red/brown marks are present in the nucleus of a cell stained with hematoxylin, and an imprinted gene is determined as having a copy number variation when more than two red/brown marks are present in the nucleus of a cell stained with hematoxylin. A copy number variation occurs when abnormal gene duplication takes place in a cancer cell such that the duplicated gene is expressed as having three or more copies of the original gene.

In this application, the hematoxylin-stained marks include but are not limited to red marks and brown marks. Staining/marking in other colors is also feasible when analyzing imprinted gene expression.

According to this application, a probe/probes specific to the imprinted gene(s) to be detected in a sample is/are used for in-situ hybridization with the sample, and tumor diagnosis is carried out by analyzing whether the imprinted gene(s) in the sample cells has/have a loss of imprinting or a copy number variation.

In one embodiment of this application, the sample is sourced from tissue and/or cells.

In this application, it is required that samples be fixed with formalin to prevent RNA degradation. The tissue samples for use may be paraffin sections, and the steps of obtaining a paraffin section include obtaining a human tumor tissue sample, fixing the tissue with 10% neutral buffered formalin in time, embedding the tissue in paraffin, cutting a 10-µm section out of the paraffin-embedded tissue, and making a tissue slide with a positively charged glass slide.

In one embodiment of this application, the in-situ hybridization uses an RNAscope in-situ hybridization method.

In this application, the probes for use in the in-situ hybridization are designed according to the sequences of the imprinted genes to be detected. More specifically, a sequence is selected from the intron of each of the to-be-detected genes as the corresponding probe. The probes used in this application were designed by Advanced Cell Diagnostics.

In one embodiment of this application, the RNAscope in-situ hybridization method uses a single- or multiple-channel chromogenic reagent kit or a single- or multiple-channel fluorescent reagent kit, preferably a single-channel red/brown chromogenic reagent kit or a multiple-channel fluorescent reagent kit.

The analysis method of the tumor diagnosis device is detailed as follows.

In one embodiment of this application, a bile duct tissue and/or cell sample is subjected to an analysis of the expression of the imprinted genes Z1, Z2, Z8, Z10, N11, Z14, N16, Z19, Z20, Z21, Z22, Z25, Z26, and Z27.

If the imprinted genes Z1, Z2, Z8, Z10, N11, Z14, N16, Z19, Z20, Z21, Z22, Z25, Z26, and Z27 are not expressed or are expressed as normal in the cell nuclei in the bile duct tissue and/or cell sample, the sample will be determined as a healthy bile duct tissue and/or cell sample.

If, however, the imprinted gene Z1, Z2, Z8, Z10, N11, Z14, N16, Z19, Z20, Z21, Z22, Z25, Z26, or Z27 is expressed as having a loss of imprinting and/or a copy number variation in the cell nuclei in the bile duct tissue and/or cell sample, the sample will be determined as a cholangiocarcinoma tissue and/or cell sample.

In one embodiment of this application, a brain tissue and/or cell sample is subjected to an analysis of the expression of the imprinted genes Z1, Z2, Z4, Z5, Z8, Z9, Z10, N11, Z13, Z14, N16, Z17, Z19, Z21, Z22, Z23, Z26, Z27, and Z28.

If the imprinted genes Z1, Z2, Z4, Z5, Z8, Z9, Z10, N11, Z13, Z14, N16, Z17, Z19, Z21, Z22, Z23, Z26, Z27, and Z28 are not expressed or are expressed as normal in the cell nuclei in the brain tissue and/or cell sample, the sample will be determined as a healthy brain tissue and/or cell sample.

If, however, the imprinted gene Z1, Z2, Z4, Z5, Z8, Z9, Z10, N11, Z13, Z14, N16, Z17, Z19, Z21, Z22, Z23, Z26, Z27, or Z28 is expressed as having a loss of imprinting and/or a copy number variation in the cell nuclei in the brain tissue and/or cell sample, the sample will be determined as a brain cancer tissue and/or cell sample.

In one embodiment of this application, a kidney tissue and/or cell sample is subjected to an analysis of the expression of the imprinted genes Z1, Z4, Z6, Z8, Z10, N11, Z13, N16, Z17, Z18, Z19, Z20, Z21, Z25, Z26, Z27, and Z28.

If the imprinted genes Z1, Z4, Z6, Z8, Z10, N11, Z13, N16, Z17, Z18, Z19, Z20, Z21, Z25, Z26, Z27, and Z28 are not expressed or are expressed as normal in the cell nuclei in the kidney tissue and/or cell sample, the sample will be determined as a healthy kidney tissue and/or cell sample.

If, however, the imprinted gene Z1, Z4, Z6, Z8, Z10, N11, Z13, N16, Z17, Z18, Z19, Z20, Z21, Z25, Z26, Z27, or Z28 is expressed as having a loss of imprinting and/or a copy number variation in the cell nuclei in the kidney tissue and/or cell sample, the sample will be determined as a kidney cancer tissue and/or cell sample.

In one embodiment of this application, a bone tissue and/or cell sample is subjected to an analysis of the expression of the imprinted genes Z1, Z2, Z3, Z6, Z8, Z9, Z10, N11, Z13, N16, Z18, Z19, Z20, Z21, Z22, Z25, Z26, Z27, and Z28.

If the imprinted genes Z1, Z2, Z3, Z6, Z8, Z9, Z10, N11, Z13, N16, Z18, Z19, Z20, Z21, Z22, Z25, Z26, Z27, and Z28 are not expressed or are expressed as normal in the cell nuclei in the bone tissue and/or cell sample, the sample will be determined as a healthy bone tissue and/or cell sample.

If, however, the imprinted gene Z1, Z2, Z3, Z6, Z8, Z9, Z10, N11, Z13, N16, Z18, Z19, Z20, Z21, Z22, Z25, Z26, Z27, or Z28 is expressed as having a loss of imprinting and/or a copy number variation in the cell nuclei in the bone tissue and/or cell sample, the sample will be determined as a bone cancer tissue and/or cell sample.

In one embodiment of this application, a cervical tissue and/or cell sample is subjected to an analysis of the expression of the imprinted genes Z1, Z3, Z5, Z8, Z9, Z10, N11, N16, Z17, Z19, Z20, Z21, Z22, Z23, Z26, Z27, and Z28.

If the imprinted genes Z1, Z3, Z5, Z8, Z9, Z10, N11, N16, Z17, Z19, Z20, Z21, Z22, Z23, Z26, Z27, and Z28 are not expressed or are expressed as normal in the cell nuclei in the cervical tissue and/or cell sample, the sample will be determined as a healthy cervical tissue and/or cell sample.

If, however, the imprinted gene Z1, Z3, Z5, Z8, Z9, Z10, N11, N16, Z17, Z19, Z20, Z21, Z22, Z23, Z26, Z27, or Z28 is expressed as having a loss of imprinting and/or a copy number variation in the cell nuclei in the cervical tissue and/or cell sample, the sample will be determined as a cervical cancer tissue and/or cell sample.

In one embodiment of this application, a nasopharyngeal tissue and/or cell sample is subjected to an analysis of the expression of the imprinted genes Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, and Z28.

If the imprinted genes Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, and Z28 are not expressed or are expressed as normal in the cell nuclei in the nasopharyngeal tissue and/or cell sample, the sample will be determined as a healthy nasopharyngeal tissue and/or cell sample.

If, however, the imprinted gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, or Z28 is expressed as having a loss of imprinting and/or a copy number variation in the cell nuclei in the nasopharyngeal tissue and/or cell sample, the sample will be determined as a nasopharyngeal carcinoma tissue and/or cell sample.

In one embodiment of this application, a laryngeal tissue and/or cell sample is subjected to an analysis of the expression of the imprinted genes Z1, Z3, Z6, Z8, N11, Z15, N16, Z17, Z18, Z19, Z21, Z22, Z25, Z26, Z27, and Z28.

If the imprinted genes Z1, Z3, Z6, Z8, N11, Z15, N16, Z17, Z18, Z19, Z21, Z22, Z25, Z26, Z27, and Z28 are not expressed or are expressed as normal in the cell nuclei in the laryngeal tissue and/or cell sample, the sample will be determined as a healthy laryngeal tissue and/or cell sample.

If, however, the imprinted gene Z1, Z3, Z6, Z8, N11, Z15, N16, Z17, Z18, Z19, Z21, Z22, Z25, Z26, Z27, or Z28 is expressed as having a loss of imprinting and/or a copy number variation in the cell nuclei in the laryngeal tissue and/or cell sample, the sample will be determined as a laryngeal cancer tissue and/or cell sample.

In one embodiment of this application, a prostate tissue and/or cell sample is subjected to an analysis of the expression of the imprinted genes Z18, Z19, Z20, Z21, Z25, Z26, Z27, and Z28.

If the imprinted genes Z18, Z19, Z20, Z21, Z25, Z26, Z27, and Z28 are not expressed or are expressed as normal in the cell nuclei in the prostate tissue and/or cell sample, the sample will be determined as a healthy prostate tissue and/or cell sample.

If, however, the imprinted gene Z18, Z19, Z20, Z21, Z25, Z26, Z27, or Z28 is expressed as having a loss of imprinting and/or a copy number variation in the cell nuclei in the prostate tissue and/or cell sample, the sample will be determined as a prostate cancer tissue and/or cell sample.

In one embodiment of this application, a gastric tissue and/or cell sample is subjected to an analysis of the expression of the imprinted genes Z18, Z19, Z20, Z21, Z22, Z26, and Z27.

If the imprinted genes Z18, Z19, Z20, Z21, Z22, Z26, and Z27 are not expressed or are expressed as normal in the cell nuclei in the gastric tissue and/or cell sample, the sample will be determined as a healthy gastric tissue and/or cell sample.

If, however, the imprinted gene Z18, Z19, Z20, Z21, Z22, Z26, or Z27 is expressed as having a loss of imprinting and/or a copy number variation in the cell nuclei in the gastric tissue and/or cell sample, the sample will be determined as a gastric carcinoma tissue and/or cell sample.

In one embodiment of this application, an esophageal tissue and/or cell sample is subjected to an analysis of the expression of the imprinted genes Z18, Z19, Z21, Z22, Z26, Z27, and Z28.

If the imprinted genes Z18, Z19, Z21, Z22, Z26, Z27, and Z28 are not expressed or are expressed as normal in the cell nuclei in the esophageal tissue and/or cell sample, the sample will be determined as a healthy esophageal tissue and/or cell sample.

If, however, the imprinted gene Z18, Z19, Z21, Z22, Z26, Z27, or Z28 is expressed as having a loss of imprinting and/or a copy number variation in the cell nuclei in the esophageal tissue and/or cell sample, the sample will be determined as an esophageal cancer tissue and/or cell sample.

In one embodiment of this application, a mammary tissue and/or cell sample is subjected to an analysis of the expression of the imprinted genes Z18, Z19, Z21, Z22, and Z27.

If the imprinted genes Z18, Z19, Z21, Z22, and Z27 are not expressed or are expressed as normal in the cell nuclei in the mammary tissue and/or cell sample, the sample will be determined as a healthy mammary tissue and/or cell sample.

If, however, the imprinted gene Z18, Z19, Z21, Z22, or Z27 is expressed as having a loss of imprinting and/or a copy number variation in the cell nuclei in the mammary tissue and/or cell sample, the sample will be determined as a mammary cancer tissue and/or cell sample.

In one embodiment of this application, a thyroid tissue and/or cell sample is subjected to an analysis of the expression of the imprinted genes Z18, Z19, Z20, Z21, and Z23.

If the imprinted genes Z18, Z19, Z20, Z21, and Z2 are not expressed or are expressed as normal in the cell nuclei in the thyroid tissue and/or cell sample, the sample will be determined as a healthy thyroid tissue and/or cell sample.

If, however, the imprinted gene Z18, Z19, Z20, Z21, or Z23 is expressed as having a loss of imprinting and/or a copy number variation in the cell nuclei in the thyroid tissue and/or cell sample, the sample will be determined as a thyroid cancer tissue and/or cell sample.

In one embodiment of this application, a bladder tissue and/or cell sample is subjected to an analysis of the expression of the imprinted genes Z18, Z19, Z20, Z21, Z22, Z26, Z27, and Z28.

If the imprinted genes Z18, Z19, Z20, Z21, Z22, Z26, Z27, and Z28 are not expressed or are expressed as normal in the cell nuclei in the bladder tissue and/or cell sample, the sample will be determined as a healthy bladder tissue and/or cell sample.

If, however, the imprinted gene Z18, Z19, Z20, Z21, Z22, Z26, Z27, or Z2 is expressed as having a loss of imprinting and/or a copy number variation in the cell nuclei in the bladder tissue and/or cell sample, the sample will be determined as a bladder cancer tissue and/or cell sample.

In one embodiment of this application, a liver tissue and/or cell sample is subjected to an analysis of the expression of the imprinted genes Z17, Z19, Z21, Z22, Z26, Z27, and Z28.

If the imprinted genes Z17, Z19, Z21, Z22, Z26, Z27, and Z28 are not expressed or are expressed as normal in the cell nuclei in the liver tissue and/or cell sample, the sample will be determined as a healthy liver tissue and/or cell sample.

If, however, the imprinted gene Z17, Z19, Z21, Z22, Z26, Z27, or Z28 is expressed as having a loss of imprinting and/or a copy number variation in the cell nuclei in the liver tissue and/or cell sample, the sample will be determined as a liver cancer tissue and/or cell sample.

In one embodiment of this application, a pancreatic tissue and/or cell sample is subjected to an analysis of the expression of the imprinted genes Z17, Z18, Z19, Z20, Z21, Z22, Z26, Z27, and Z28.

If the imprinted genes Z17, Z18, Z19, Z20, Z21, Z22, Z26, Z27, and Z28 are not expressed or are expressed as normal in the cell nuclei in the pancreatic tissue and/or cell sample, the sample will be determined as a healthy pancreatic tissue and/or cell sample.

If, however, the imprinted gene Z17, Z18, Z19, Z20, Z21, Z22, Z26, Z27, or Z28 is expressed as having a loss of imprinting and/or a copy number variation in the cell nuclei in the pancreatic tissue and/or cell sample, the sample will be determined as a pancreatic cancer tissue and/or cell sample.

In one embodiment of this application, a lymphatic tissue and/or cell sample is subjected to an analysis of the expression of the imprinted genes Z18, Z19, Z21, Z22, Z26, Z27, and Z28.

If the imprinted genes Z18, Z19, Z21, Z22, Z26, Z27, and Z28 are not expressed or are expressed as normal in the cell nuclei in the lymphatic tissue and/or cell sample, the sample will be determined as a healthy lymphatic tissue and/or cell sample.

If, however, the imprinted gene Z18, Z19, Z21, Z22, Z26, Z27, or Z28 is expressed as having a loss of imprinting and/or a copy number variation in the cell nuclei in the lymphatic tissue and/or cell sample, the sample will be determined as a lymphoma tissue and/or cell sample.

In one embodiment of this application, a colorectal tissue and/or cell sample is subjected to an analysis of the expression of the imprinted genes Z19, Z20, Z21, Z22, Z26, and Z27.

If the imprinted genes Z19, Z20, Z21, Z22, Z26, and Z27 are not expressed or are expressed as normal in the cell nuclei in the colorectal tissue and/or cell sample, the sample will be determined as a healthy colorectal tissue and/or cell sample.

If, however, the imprinted gene Z19, Z20, Z21, Z22, Z26, or Z27 is expressed as having a loss of imprinting and/or a copy number variation in the cell nuclei in the colorectal tissue and/or cell sample, the sample will be determined as a colorectal cancer tissue and/or cell sample.

In one embodiment of this application, a skin tissue and/or cell sample is subjected to an analysis of the expression of the imprinted genes Z18, Z19, Z21, Z22, Z25, and Z28.

If the imprinted genes Z18, Z19, Z21, Z22, Z25, and Z28 are not expressed or are expressed as normal in the cell nuclei in the skin tissue and/or cell sample, the sample will be determined as a healthy skin tissue and/or cell sample.

If, however, the imprinted gene Z18, Z19, Z21, Z22, Z25, or Z28 is expressed as having a loss of imprinting and/or a copy number variation in the cell nuclei in the skin tissue and/or cell sample, the sample will be determined as a skin cancer tissue and/or cell sample.

In one embodiment of this application, a lung tissue and/or cell sample is subjected to an analysis of the expression of the imprinted genes Z18, Z19, Z21, Z22, Z25, Z26, Z27, and Z28.

If the imprinted genes Z18, Z19, Z21, Z22, Z25, Z26, Z27, and Z28 are not expressed or are expressed as normal in the cell nuclei in the lung tissue and/or cell sample, the sample will be determined as a healthy lung tissue and/or cell sample.

If, however, the imprinted gene Z18, Z19, Z21, Z22, Z25, Z26, Z27, or Z28 is expressed as having a loss of imprinting and/or a copy number variation in the cell nuclei in the lung tissue and/or cell sample, the sample will be determined as a lung cancer tissue and/or cell sample.

This application has the following advantageous effects over the prior art:
(1) The imprinted genes in this application are not expressed or are expressed as normal in normal tissue or cells, show a loss of imprinting or a copy number variation in a large number of cells in cancer tissue, and therefore have significant diagnostic sensitivity when used as tumor markers.
(2) The imprinted gene-based tumor markers in this application can provide guidance on tumor treatment, facilitate early diagnosis of tumors, help increase patients' survival rates, and thus contribute greatly to saving patients' lives.
(3) The detection method in this application is different from immunohistochemistry, DNA sequencing, DNA methylation analysis, and fluorescent in-situ hybridization (FISH) in that it can reduce false positives and other negative effects while providing important guidance on the treatment and medication of tumors.

### Brief Description of the Several Views of the Drawings

FIG 1 shows how 14 imprinted genes are expressed in bile duct tissue samples, with FIG. 1(a) showing how the 14 imprinted genes are expressed in a healthy bile duct tissue sample, and FIG. 1(b) showing how the 14 imprinted genes are expressed in a cholangiocarcinoma tissue sample;
FIG 2 shows how 19 imprinted genes are expressed in brain tissue samples, with FIG. 2(a) showing how the 19 imprinted genes are expressed in a healthy brain tissue sample, and FIG. 2(b) showing how the 19 imprinted genes are expressed in a brain cancer tissue sample;
FIG 3 shows how 17 imprinted genes are expressed in kidney tissue samples, with FIG. 3(a) showing how the 17 imprinted genes are expressed in a healthy kidney tissue sample, and FIG. 3(b) showing how the 17 imprinted genes are expressed in a kidney cancer tissue sample;
FIG 4 shows how 19 imprinted genes are expressed in bone tissue samples, with FIG. 4(a) showing how the 19 imprinted genes are expressed in a healthy bone tissue sample, and FIG. 4(b) showing how the 19 imprinted genes are expressed in a bone cancer tissue sample;
FIG 5 shows how 17 imprinted genes are expressed in cervical tissue samples, with FIG. 5(a) showing how the 17 imprinted genes are expressed in a healthy cervical tissue sample, and FIG. 5(b) showing how the 17 imprinted genes are expressed in a cervical cancer tissue sample;
FIG 6 shows how 24 imprinted genes are expressed in nasopharyngeal tissue samples, with FIG. 6(a) showing how the 24 imprinted genes are expressed in a healthy nasopharyngeal tissue sample, and FIG. 6(b) showing how the 24 imprinted genes are expressed in a nasopharyngeal carcinoma tissue sample;
FIG 7 shows how 16 imprinted genes are expressed in laryngeal tissue samples, with FIG. 7(a) showing how the 16 imprinted genes are expressed in a healthy laryngeal tissue sample, and FIG. 7(b) showing how the 16 imprinted genes are expressed in a laryngeal cancer tissue sample;
FIG 8 shows how 8 imprinted genes are expressed in prostate tissue samples, with FIG. 8(a) showing how the 8 imprinted genes are expressed in a healthy prostate tissue sample, and FIG. 8(b) showing how the 8 imprinted genes are expressed in a prostate cancer tissue sample;
FIG 9 shows how 7 imprinted genes are expressed in gastric tissue samples, with FIG. 9(a) showing how the 7 imprinted genes are expressed in a healthy gastric tissue sample, and FIG. 9(b) showing how the 7 imprinted genes are expressed in a gastric carcinoma tissue sample;
FIG 10 shows how 7 imprinted genes are expressed in esophageal tissue samples, with FIG. 10(a) showing how the 7 imprinted genes are expressed in a healthy esophageal tissue sample, and FIG. 10(b) showing how the 7 imprinted genes are expressed in an esophageal cancer tissue sample;
FIG 11 shows how 5 imprinted genes are expressed in mammary tissue samples, with FIG. 11(a) showing how the 5 imprinted genes are expressed in a healthy mammary tissue sample, and FIG. 11(b) showing how the 5 imprinted genes are expressed in a mammary cancer tissue sample;
FIG 12 shows how 5 imprinted genes are expressed in thyroid tissue samples, with FIG. 12(a) showing how the 5 imprinted genes are expressed in a healthy thyroid tissue sample, and FIG. 12(b) showing how the 5 imprinted genes are expressed in a thyroid cancer tissue sample;
FIG 13 shows how 8 imprinted genes are expressed in bladder tissue samples, with FIG. 13(a) showing how the 8 imprinted genes are expressed in a healthy bladder tissue sample, and FIG. 13(b) showing how the 8 imprinted genes are expressed in a bladder cancer tissue sample;
FIG 14 shows how 7 imprinted genes are expressed in liver tissue samples, with FIG. 14(a) showing how the 7 imprinted genes are expressed in a healthy liver tissue sample, and FIG. 14(b) showing how the 7 imprinted genes are expressed in a liver cancer tissue sample;
FIG 15 shows how 9 imprinted genes are expressed in pancreatic tissue samples, with FIG. 15(a) showing how the 9 imprinted genes are expressed in a healthy pancreatic tissue sample, and FIG. 15(b) showing how the 9 imprinted genes are expressed in a pancreatic cancer tissue sample;
FIG 16 shows how 7 imprinted genes are expressed in lymphatic tissue samples, with FIG. 16(a) showing how the 7 imprinted genes are expressed in a healthy lymphatic tissue sample, and FIG. 16(b) showing how the 7 imprinted genes are expressed in a lymphoma tissue sample;
FIG 17 shows how 6 imprinted genes are expressed in colorectal tissue samples, with FIG. 17(a) showing how the 6 imprinted genes are expressed in a healthy colorectal tissue sample, and FIG. 17(b) showing how the 6 imprinted genes are expressed in a colorectal cancer tissue sample;
FIG 18 shows how 6 imprinted genes are expressed in skin tissue samples, with FIG. 18(a) showing how the 6 imprinted genes are expressed in a healthy skin tissue sample, and FIG. 18(b) showing how the 6 imprinted genes are expressed in a skin cancer tissue sample; and
FIG 19 shows how 8 imprinted genes are expressed in lung tissue samples, with FIG. 19(a) showing how the 8 imprinted genes are expressed in a healthy lung tissue sample, and FIG. 19(b) showing how the 8 imprinted genes are expressed in a lung cancer tissue sample.

### Detailed Description of the Invention

To further explain the technical means used in this application and its effects, certain embodiments are detailed below with reference to the accompanying drawings. It should be understood that the embodiments described herein serve only to expound, but not to limit the scope of, the subject matter of this application.

While some of the techniques or conditions used in the embodiments may not be specified herein, those techniques or conditions can be implemented according to the corresponding description in literature in the same technical field as this application or according to the corresponding product manuals. Any reagent or instrument that was used in the embodiments but manufacturer of which is not specified herein is a conventional product commercially available through regular marketing channels.

### Embodiment 1: imprinted gene analysis on cholangiocarcinoma samples

(1) Healthy bile duct tissue and cholangiocarcinoma tissue were obtained and placed in 10% neutral buffered formalin in order to be fixed, lest the RNA degrade. After fixing for 24 hours and embedment in paraffin (FFPE), the embedded tissues were cut into 10 µm-thick sections, which were loaded onto positively charged slides. The slides were then baked in a 40°C oven for at least 3 hours.
(2) The sample processing methods of RNAscope were used to dewax the sections, block the activity of the endogenous peroxidase in the samples, permeabilize the tissues, and expose the RNA molecules.
(3) Probe design: Specific probes were designed according to the sequences of the imprinted genes Z1, Z2, Z8, Z10, N11, Z14, N16, Z19, Z20, Z21, Z22, Z25, Z26, and Z27.
(4) RNAscope reagent kits were used to perform in-situ hybridization between the probes in step (3) and the samples.
(5) After staining with hematoxylin for signal amplification, expression of the imprinted genes was analyzed via microscopic imaging.

In this embodiment, expression of the aforesaid imprinted genes included an imprinted gene not being expressed, the imprinted gene being expressed as normal, the imprinted gene being expressed as having a loss of imprinting, and the imprinted gene being expressed as having a copy number variation, wherein: an imprinted gene was determined as not being expressed when no imprinted gene mark was present in the nucleus of a sample cell, an imprinted gene was determined as being expressed as normal when only one imprinted gene mark was present in the nucleus of a sample cell, an imprinted gene was determined as having a loss of imprinting when two imprinted gene marks were present in the nucleus of a sample cell, and an imprinted gene was determined as having a copy number variation when more than two imprinted gene marks were present in the nucleus of a sample cell.

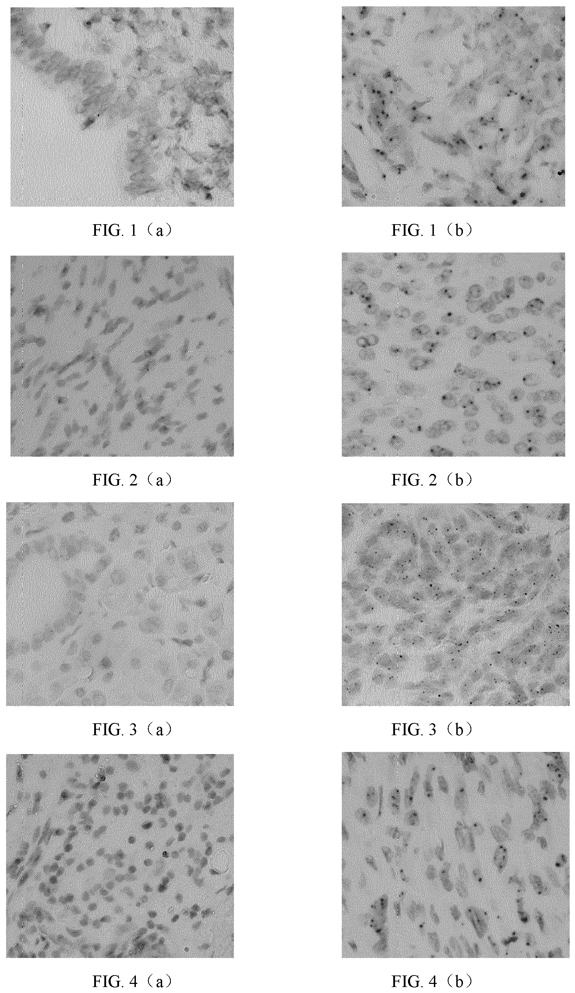

As shown in FIG. 1(a), the 14 imprinted genes were not expressed or were expressed as normal in a healthy bile duct tissue sample, with only a small number of individual cells showing a loss of imprinting or a copy number variation. In a cholangiocarcinoma tissue sample, however, loss of imprinting and copy number variation were found in a large number of cells as shown in FIG. 1(b).

### Embodiment 2: imprinted gene analysis on brain cancer samples

Healthy brain tissue samples and brain cancer tissue samples were obtained, and FFPE sections of the samples were prepared. Specific probes were designed according to the sequences of the imprinted genes Z1, Z2, Z4, Z5, Z8, Z9, Z10, N11, Z13, Z14, N16, Z17, Z19, Z21, Z22, Z23, Z26, Z27, and Z28. The remaining steps of the analysis were the same as those in embodiment 1.

As shown in FIG. 2(a), the 19 imprinted genes were not expressed or were expressed as normal in a healthy brain tissue sample, with only a small number of individual cells showing a loss of imprinting or a copy number variation. In a brain cancer tissue sample, however, loss of imprinting and copy number variation were found in a large number of cells as shown in FIG. 2(b).

### Embodiment 3: imprinted gene analysis on kidney cancer samples

Healthy kidney tissue samples and kidney cancer tissue samples were obtained, and FFPE sections of the samples were prepared. Specific probes were designed according to the sequences of the imprinted genes Z1, Z4, Z6, Z8, Z10, N11, Z13, N16, Z17, Z18, Z19, Z20, Z21, Z25, Z26, Z27, and Z28. The remaining steps of the analysis were the same as those in embodiment 1.

As shown in FIG. 3(a), the 17 imprinted genes were not expressed or were expressed as normal in a healthy kidney tissue sample, with only a small number of individual cells showing a loss of imprinting or a copy number variation. In a kidney cancer tissue sample, however, loss of imprinting and copy number variation were found in a large number of cells as shown in FIG. 3(b).

### Embodiment 4: imprinted gene analysis on bone cancer samples

Healthy bone tissue samples and bone cancer tissue samples were obtained, and FFPE sections of the samples were prepared. Specific probes were designed according to the sequences of the imprinted genes Z1, Z2, Z3, Z6, Z8, Z9, Z10, N11, Z13, N16, Z18, Z19, Z20, Z21, Z22, Z25, Z26, Z27, and Z28. The remaining steps of the analysis were the same as those in embodiment 1.

As shown in FIG. 4(a), the 19 imprinted genes were not expressed or were expressed as normal in a healthy bone tissue sample, with only a small number of individual cells showing a loss of imprinting or a copy number variation. In a bone cancer tissue sample, however, loss of imprinting and copy number variation were found in a large number of cells as shown in FIG. 4(b).

### Embodiment 5: imprinted gene analysis on cervical cancer samples

Healthy cervical tissue samples and cervical cancer tissue samples were obtained, and FFPE sections of the samples were prepared. Specific probes were designed according to the sequences of the imprinted genes Z1, Z3, Z5, Z8, Z9, Z10, N11, N16, Z17, Z19, Z20, Z21, Z22, Z23, Z26, Z27, and Z28. The remaining steps of the analysis were the same as those in embodiment 1.

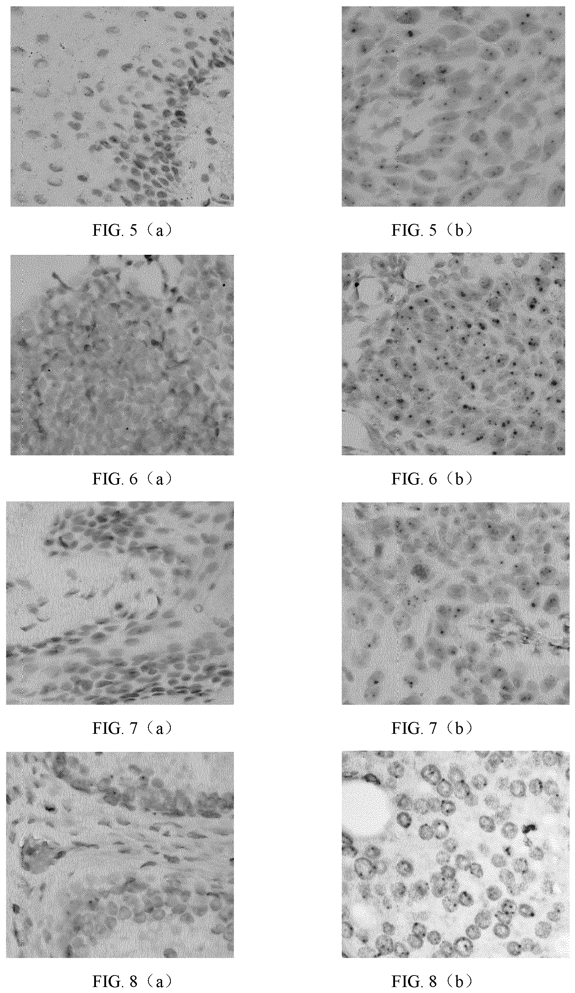

As shown in FIG. 5(a), the 17 imprinted genes were not expressed or were expressed as normal in a healthy cervical tissue sample, with only a small number of individual cells showing a loss of imprinting or a copy number variation. In a cervical cancer tissue sample, however, loss of imprinting and copy number variation were found in a large number of cells as shown in FIG. 5(b).

### Embodiment 6: imprinted gene analysis on nasopharyngeal carcinoma samples

Healthy nasopharyngeal tissue samples and nasopharyngeal carcinoma tissue samples were obtained, and FFPE sections of the samples were prepared. Specific probes were designed according to the sequences of the imprinted genes Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, and Z28. The remaining steps of the analysis were the same as those in embodiment 1.

As shown in FIG. 6(a), the 24 imprinted genes were not expressed or were expressed as normal in a healthy nasopharyngeal tissue sample, with only a small number of individual cells showing a loss of imprinting or a copy number variation. In a nasopharyngeal carcinoma tissue sample, however, loss of imprinting and copy number variation were found in a large number of cells as shown in FIG. 6(b).

### Embodiment 7: imprinted gene analysis on laryngeal cancer samples

Healthy laryngeal tissue samples and laryngeal cancer tissue samples were obtained, and FFPE sections of the samples were prepared. Specific probes were designed according to the sequences of the imprinted genes Z1, Z3, Z6, Z8, N11, Z15, N16, Z17, Z18, Z19, Z21, Z22, Z25, Z26, Z27, and Z28. The remaining steps of the analysis were the same as those in embodiment 1.

As shown in FIG. 7(a), the 16 imprinted genes were not expressed or were expressed as normal in a healthy laryngeal tissue sample, with only a small number of individual cells showing a loss of imprinting or a copy number variation. In a laryngeal cancer tissue sample, however, loss of imprinting and copy number variation were found in a large number of cells as shown in FIG. 7(b).

### Embodiment 8: imprinted gene analysis on prostate cancer samples

Healthy prostate tissue samples and prostate cancer tissue samples were obtained, and FFPE sections of the samples were prepared. Specific probes were designed according to the sequences of the imprinted genes Z18, Z19, Z20, Z21, Z25, Z26, Z27, and Z28. The remaining steps of the analysis were the same as those in embodiment 1.

As shown in FIG. 8(a), the 8 imprinted genes were not expressed or were expressed as normal in a healthy prostate tissue sample, with only a small number of individual cells showing a loss of imprinting or a copy number variation. In a prostate cancer tissue sample, however, loss of imprinting and copy number variation were found in a large number of cells as shown in FIG. 8(b).

### Embodiment 9: imprinted gene analysis on gastric carcinoma samples

Healthy gastric tissue samples and gastric carcinoma tissue samples were obtained, and FFPE sections of the samples were prepared. Specific probes were designed according to the sequences of the imprinted genes Z18, Z19, Z20, Z21, Z22, Z26, and Z27. The remaining steps of the analysis were the same as those in embodiment 1.

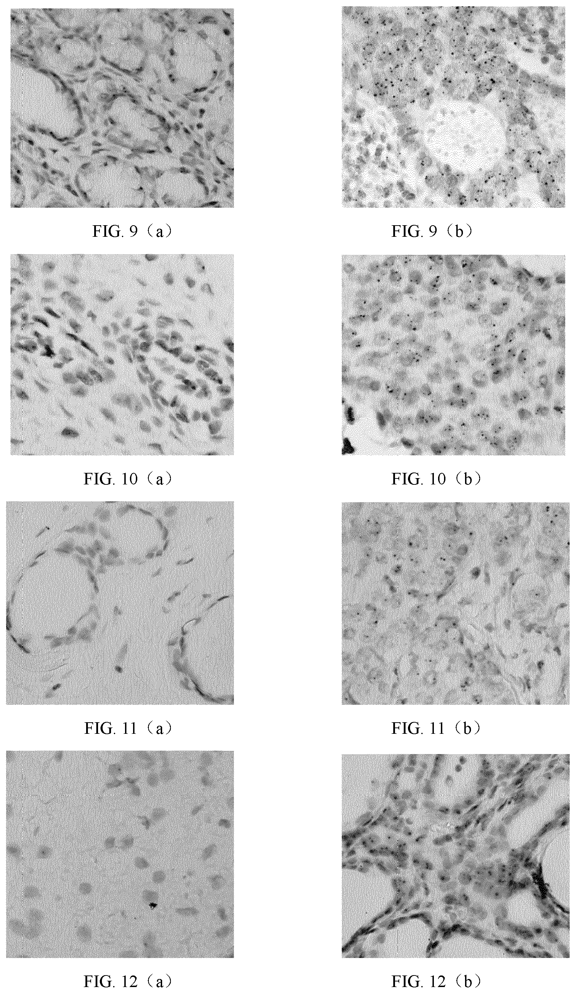

As shown in FIG. 9(a), the 7 imprinted genes were not expressed or were expressed as normal in a healthy gastric tissue sample, with only a small number of individual cells showing a loss of imprinting or a copy number variation. In a gastric carcinoma tissue sample, however, loss of imprinting and copy number variation were found in a large number of cells as shown in FIG. 9(b).

### Embodiment 10: imprinted gene analysis on esophageal cancer samples

Healthy esophageal tissue samples and esophageal cancer tissue samples were obtained, and FFPE sections of the samples were prepared. Specific probes were designed according to the sequences of the imprinted genes Z18, Z19, Z21, Z22, Z26, Z27, and Z28. The remaining steps of the analysis were the same as those in embodiment 1.

As shown in FIG. 10(a), the 7 imprinted genes were not expressed or were expressed as normal in a healthy esophageal tissue sample, with only a small number of individual cells showing a loss of imprinting or a copy number variation. In an esophageal cancer tissue sample, however, loss of imprinting and copy number variation were found in a large number of cells as shown in FIG. 10(b).

### Embodiment 11: imprinted gene analysis on mammary cancer samples

Healthy mammary tissue samples and mammary cancer tissue samples were obtained, and FFPE sections of the samples were prepared. Specific probes were designed according to the sequences of the imprinted genes Z18, Z19, Z21, Z22, and Z27. The remaining steps of the analysis were the same as those in embodiment 1.

As shown in FIG. 11(a), the 5 imprinted genes were not expressed or were expressed as normal in a healthy mammary tissue sample, with only a small number of individual cells showing a loss of imprinting or a copy number variation. In a mammary cancer tissue sample, however, loss of imprinting and copy number variation were found in a large number of cells as shown in FIG. 11(b).

### Embodiment 12: imprinted gene analysis on thyroid cancer samples

Healthy thyroid tissue samples and thyroid cancer tissue samples were obtained, and FFPE sections of the samples were prepared. Specific probes were designed according to the sequences of the imprinted genes Z18, Z19, Z20, Z21, and Z23. The remaining steps of the analysis were the same as those in embodiment 1.

As shown in FIG. 12(a), the 5 imprinted genes were not expressed or were expressed as normal in a healthy thyroid tissue sample, with only a small number of individual cells showing a loss of imprinting or a copy number variation. In a thyroid cancer tissue sample, however, loss of imprinting and copy number variation were found in a large number of cells as shown in FIG. 12(b).

### Embodiment 13: imprinted gene analysis on bladder cancer samples

Healthy bladder tissue samples and bladder cancer tissue samples were obtained, and FFPE sections of the samples were prepared. Specific probes were designed according to the sequences of the imprinted genes Z18, Z19, Z20, Z21, Z22, Z26, Z27, and Z28. The remaining steps of the analysis were the same as those in embodiment 1.

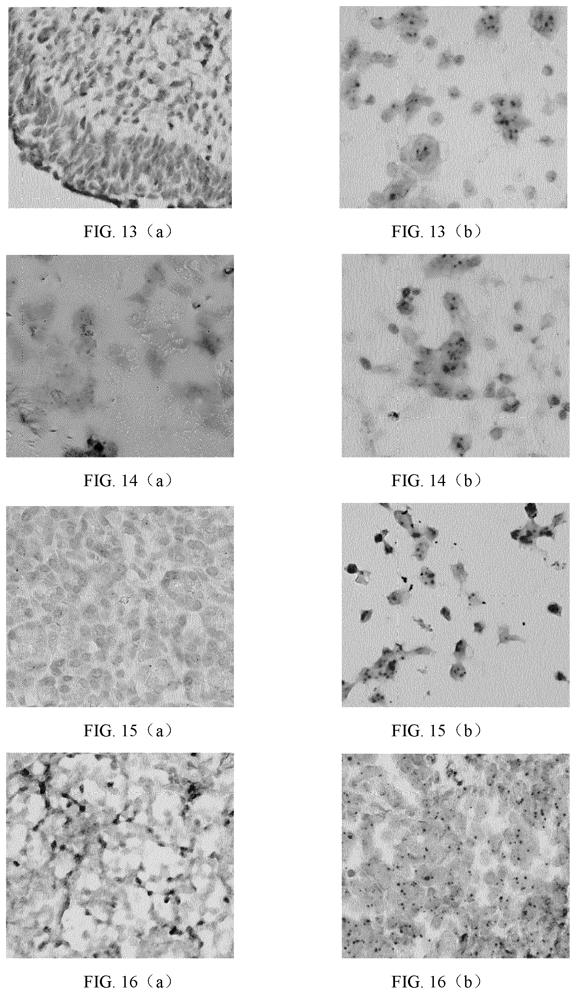

As shown in FIG. 13(a), the 8 imprinted genes were not expressed or were expressed as normal in a healthy bladder tissue sample, with only a small number of individual cells showing a loss of imprinting or a copy number variation. In a bladder cancer tissue sample, however, loss of imprinting and copy number variation were found in a large number of cells as shown in FIG. 13(b).

### Embodiment 14: imprinted gene analysis on liver cancer samples

Healthy liver tissue samples and liver cancer tissue samples were obtained, and FFPE sections of the samples were prepared. Specific probes were designed according to the sequences of the imprinted genes Z17, Z19, Z21, Z22, Z26, Z27, and Z28. The remaining steps of the analysis were the same as those in embodiment 1.

As shown in FIG. 14(a), the 7 imprinted genes were not expressed or were expressed as normal in a healthy liver tissue sample, with only a small number of individual cells showing a loss of imprinting or a copy number variation. In a liver cancer tissue sample, however, loss of imprinting and copy number variation were found in a large number of cells as shown in FIG. 14(b).

### Embodiment 15: imprinted gene analysis on pancreatic cancer samples

Healthy pancreatic tissue samples and pancreatic cancer tissue samples were obtained, and FFPE sections of the samples were prepared. Specific probes were designed according to the sequences of the imprinted genes Z17, Z18, Z19, Z20, Z21, Z22, Z26, Z27, and Z28. The remaining steps of the analysis were the same as those in embodiment 1.

As shown in FIG. 15(a), the 9 imprinted genes were not expressed or were expressed as normal in a healthy pancreatic tissue sample, with only a small number of individual cells showing a loss of imprinting or a copy number variation. In a pancreatic cancer tissue sample, however, loss of imprinting and copy number variation were found in a large number of cells as shown in FIG. 15(b).

### Embodiment 16: imprinted gene analysis on lymphoma samples

Healthy lymphatic tissue samples and lymphoma tissue samples were obtained, and FFPE sections of the samples were prepared. Specific probes were designed according to the sequences of the imprinted genes Z18, Z19, Z21, Z22, Z26, Z27, and Z28. The remaining steps of the analysis were the same as those in embodiment 1.

As shown in FIG. 16(a), the 7 imprinted genes were not expressed or were expressed as normal in a healthy lymphatic tissue sample, with only a small number of individual cells showing a loss of imprinting or a copy number variation. In a lymphoma tissue sample, however, loss of imprinting and copy number variation were found in a large number of cells as shown in FIG. 16(b).

### Embodiment 17: imprinted gene analysis on colorectal cancer samples

Healthy colorectal tissue samples and colorectal cancer tissue samples were obtained, and FFPE sections of the samples were prepared. Specific probes were designed according to the sequences of the imprinted genes Z19, Z20, Z21, Z22, Z26, and Z27. The remaining steps of the analysis were the same as those in embodiment 1.

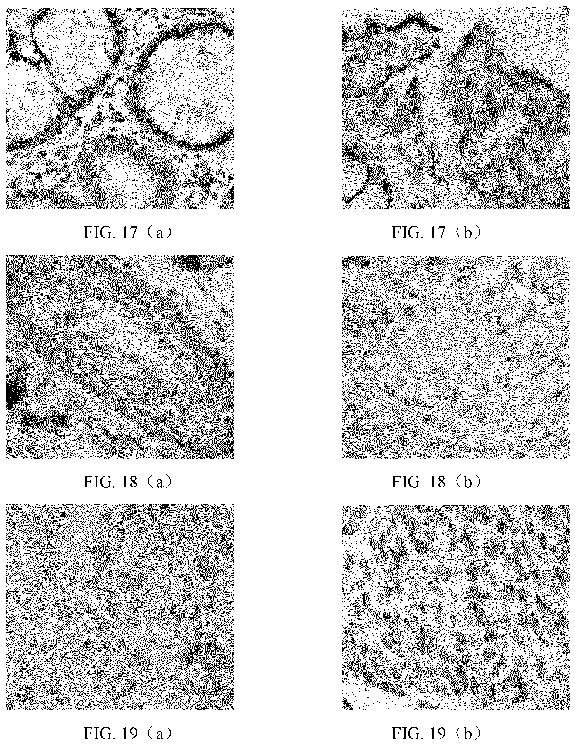

As shown in FIG. 17(a), the 6 imprinted genes were not expressed or were expressed as normal in a healthy colorectal tissue sample, with only a small number of individual cells showing a loss of imprinting or a copy number variation. In a colorectal cancer tissue sample, however, loss of imprinting and copy number variation were found in a large number of cells as shown in FIG. 17(b).

### Embodiment 18: imprinted gene analysis on skin cancer samples

Healthy skin tissue samples and skin cancer tissue samples were obtained, and FFPE sections of the samples were prepared. Specific probes were designed according to the sequences of the imprinted genes Z18, Z19, Z21, Z22, Z25, and Z28. The remaining steps of the analysis were the same as those in embodiment 1.

As shown in FIG. 18(a), the 6 imprinted genes were not expressed or were expressed as normal in a healthy skin tissue sample, with only a small number of individual cells showing a loss of imprinting or a copy number variation. In a skin cancer tissue sample, however, loss of imprinting and copy number variation were found in a large number of cells as shown in FIG. 18(b).

### Embodiment 19: imprinted gene analysis on lung cancer samples

Healthy lung tissue samples and lung cancer tissue samples were obtained, and FFPE sections of the samples were prepared. Specific probes were designed according to the sequences of the imprinted genes Z18, Z19, Z21, Z22, Z25, Z26, Z27, and Z28. The remaining steps of the analysis were the same as those in embodiment 1.

As shown in FIG. 19(a), the 8 imprinted genes were not expressed or were expressed as normal in a healthy lung tissue sample, with only a small number of individual cells showing a loss of imprinting or a copy number variation. In a lung cancer tissue sample, however, loss of imprinting and copy number variation were found in a large number of cells as shown in FIG. 19(b).

According to the above, the imprinted genes in this application are not expressed or are expressed as normal in normal tissue or cells, show a loss of imprinting or a copy number variation in a large number of cells in cancer tissue, and therefore have significant diagnostic sensitivity when used as tumor markers. Those imprinted gene-based tumor markers can provide guidance on tumor treatment, facilitate early diagnosis of tumors, help increase patients' survival rates, and thus contribute greatly to saving patients' lives.

## Claims

1. A method for tumor diagnosis comprising:
(a) subjecting a bile duct tissue and/or cell sample to an analysis of the expression of the imprinted genes Z1, Z2, Z8, Z10, N11, Z14, N16, Z19, Z20, Z21, Z22, Z25, Z26, and Z27 so that:
if the imprinted genes Z1, Z2, Z8, Z10, N11, Z14, N16, Z19, Z20, Z21, Z22, Z25, Z26, and Z27 are not expressed or are expressed as normal in cell nuclei in the bile duct tissue and/or cell sample, the bile duct tissue and/or cell sample is determined as a healthy bile duct tissue and/or cell sample; and
if the imprinted gene Z1, Z2, Z8, Z10, N11, Z14, N16, Z19, Z20, Z21, Z22, Z25, Z26, or Z27 is expressed as having a loss of imprinting and/or a copy number variation in the cell nuclei in the bile duct tissue and/or cell sample, the bile duct tissue and/or cell sample is determined as a cholangiocarcinoma tissue and/or cell sample;
or
(b) subjecting a brain tissue and/or cell sample to an analysis of the expression of the imprinted genes Z1, Z2, Z4, Z5, Z8, Z9, Z10, N11, Z13, Z14, N16, Z17, Z19, Z21, Z22, Z23, Z26, Z27, and Z28 so that:
if the imprinted genes Z1, Z2, Z4, Z5, Z8, Z9, Z10, N11, Z13, Z14, N16, Z17, Z19, Z21, Z22, Z23, Z26, Z27, and Z28 are not expressed or are expressed as normal in cell nuclei in the brain tissue and/or cell sample, the brain tissue and/or cell sample is determined as a healthy brain tissue and/or cell sample; and
if the imprinted gene Z1, Z2, Z4, Z5, Z8, Z9, Z10, N11, Z13, Z14, N16, Z17, Z19, Z21, Z22, Z23, Z26, Z27, or Z28 is expressed as having a loss of imprinting and/or a copy number variation in the cell nuclei in the brain tissue and/or cell sample, the brain tissue and/or cell sample is determined as a brain cancer tissue and/or cell sample;
or
(c) subjecting a kidney tissue and/or cell sample to an analysis of the expression of the imprinted genes Z1, Z4, Z6, Z8, Z10, N11, Z13, N16, Z17, Z18, Z19, Z20, Z21, Z25, Z26, Z27, and Z28 so that:
if the imprinted genes Z1, Z4, Z6, Z8, Z10, N11, Z13, N16, Z17, Z18, Z19, Z20, Z21, Z25, Z26, Z27, and Z28 are not expressed or are expressed as normal in cell nuclei in the kidney tissue and/or cell sample, the kidney tissue and/or cell sample is determined as a healthy kidney tissue and/or cell sample; and
if the imprinted gene Z1, Z4, Z6, Z8, Z10, N11, Z13, N16, Z17, Z18, Z19, Z20, Z21, Z25, Z26, Z27, or Z28 is expressed as having a loss of imprinting and/or a copy number variation in the cell nuclei in the kidney tissue and/or cell sample, the kidney tissue and/or cell sample is determined as a kidney cancer tissue and/or cell sample;
or
(d) subjecting a bone tissue and/or cell sample to an analysis of the expression of the imprinted genes Z1, Z2, Z3, Z6, Z8, Z9, Z10, N11, Z13, N16, Z18, Z19, Z20, Z21, Z22, Z25, Z26, Z27, and Z28 so that:
if the imprinted genes Z1, Z2, Z3, Z6, Z8, Z9, Z10, N11, Z13, N16, Z18, Z19, Z20, Z21, Z22, Z25, Z26, Z27, and Z28 are not expressed or are expressed as normal in cell nuclei in the bone tissue and/or cell sample, the bone tissue and/or cell sample is determined as a healthy bone tissue and/or cell sample; and
if the imprinted gene Z1, Z2, Z3, Z6, Z8, Z9, Z10, N11, Z13, N16, Z18, Z19, Z20, Z21, Z22, Z25, Z26, Z27, or Z28 is expressed as having a loss of imprinting and/or a copy number variation in the cell nuclei in the bone tissue and/or cell sample, the bone tissue and/or cell sample is determined as a bone cancer tissue and/or cell sample;
or
(e) subjecting a cervical tissue and/or cell sample to an analysis of the expression of the imprinted genes Z1, Z3, Z5, Z8, Z9, Z10, N11, N16, Z17, Z19, Z20, Z21, Z22, Z23, Z26, Z27, and Z28 so that:
if the imprinted genes Z1, Z3, Z5, Z8, Z9, Z10, N11, N16, Z17, Z19, Z20, Z21, Z22, Z23, Z26, Z27, and Z28 are not expressed or are expressed as normal in cell nuclei in the cervical tissue and/or cell sample, the cervical tissue and/or cell sample is determined as a healthy cervical tissue and/or cell sample; and
if the imprinted gene Z1, Z3, Z5, Z8, Z9, Z10, N11, N16, Z17, Z19, Z20, Z21, Z22, Z23, Z26, Z27, or Z28 is expressed as having a loss of imprinting and/or a copy number variation in the cell nuclei in the cervical tissue and/or cell sample, the cervical tissue and/or cell sample is determined as a cervical cancer tissue and/or cell sample;
or
(f) subjecting a nasopharyngeal tissue and/or cell sample to an analysis of the expression of the imprinted genes Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, and Z28 so that:
if the imprinted genes Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, and Z28 are not expressed or are expressed as normal in cell nuclei in the nasopharyngeal tissue and/or cell sample, the nasopharyngeal tissue and/or cell sample is determined as a healthy nasopharyngeal tissue and/or cell sample; and
if the imprinted gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, or Z28 is expressed as having a loss of imprinting and/or a copy number variation in the cell nuclei in the nasopharyngeal tissue and/or cell sample, the nasopharyngeal tissue and/or cell sample is determined as a nasopharyngeal carcinoma tissue and/or cell sample;
or
(g) subjecting a laryngeal tissue and/or cell sample to an analysis of the expression of the imprinted genes Z1, Z3, Z6, Z8, N11, Z15, N16, Z17, Z18, Z19, Z21, Z22, Z25, Z26, Z27, and Z28 so that:
if the imprinted genes Z1, Z3, Z6, Z8, N11, Z15, N16, Z17, Z18, Z19, Z21, Z22, Z25, Z26, Z27, and Z28 are not expressed or are expressed as normal in cell nuclei in the laryngeal tissue and/or cell sample, the laryngeal tissue and/or cell sample is determined as a healthy laryngeal tissue and/or cell sample; and
if the imprinted gene Z1, Z3, Z6, Z8, N11, Z15, N16, Z17, Z18, Z19, Z21, Z22, Z25, Z26, Z27, or Z28 is expressed as having a loss of imprinting and/or a copy number variation in the cell nuclei in the laryngeal tissue and/or cell sample, the laryngeal tissue and/or cell sample is determined as a laryngeal cancer tissue and/or cell sample;
or
(h) subjecting a prostate tissue and/or cell sample to an analysis of the expression of the imprinted genes Z18, Z19, Z20, Z21, Z25, Z26, Z27, and Z28 so that:
if the imprinted genes Z18, Z19, Z20, Z21, Z25, Z26, Z27, and Z28 are not expressed or are expressed as normal in cell nuclei in the prostate tissue and/or cell sample, the prostate tissue and/or cell sample is determined as a healthy prostate tissue and/or cell sample; and
if the imprinted gene Z18, Z19, Z20, Z21, Z25, Z26, Z27, or Z28 is expressed as having a loss of imprinting and/or a copy number variation in the cell nuclei in the prostate tissue and/or cell sample, the prostate tissue and/or cell sample is determined as a prostate cancer tissue and/or cell sample;
or
(i) subjecting a gastric tissue and/or cell sample to an analysis of the expression of the imprinted genes Z18, Z19, Z20, Z21, Z22, Z26, and Z27 so that:
if the imprinted genes Z18, Z19, Z20, Z21, Z22, Z26, and Z27 are not expressed or are expressed as normal in cell nuclei in the gastric tissue and/or cell sample, the gastric tissue and/or cell sample is determined as a healthy gastric tissue and/or cell sample; and
if the imprinted gene Z18, Z19, Z20, Z21, Z22, Z26, or Z27 is expressed as having a loss of imprinting and/or a copy number variation in the cell nuclei in the gastric tissue and/or cell sample, the gastric tissue and/or cell sample is determined as a gastric carcinoma tissue and/or cell sample;
or
(j) subjecting an esophageal tissue and/or cell sample to an analysis of the expression of the imprinted genes Z18, Z19, Z21, Z22, Z26, Z27, and Z28 so that:
if the imprinted genes Z18, Z19, Z21, Z22, Z26, Z27, and Z28 are not expressed or are expressed as normal in cell nuclei in the esophageal tissue and/or cell sample, the esophageal tissue and/or cell sample is determined as a healthy esophageal tissue and/or cell sample; and
if the imprinted gene Z18, Z19, Z21, Z22, Z26, Z27, or Z28 is expressed as having a loss of imprinting and/or a copy number variation in the cell nuclei in the esophageal tissue and/or cell sample, the esophageal tissue and/or cell sample is determined as an esophageal cancer tissue and/or cell sample;
or
(k) subjecting a mammary tissue and/or cell sample to an analysis of the expression of the imprinted genes Z18, Z19, Z21, Z22, and Z27 so that:
if the imprinted genes Z18, Z19, Z21, Z22, and Z27 are not expressed or are expressed as normal in cell nuclei in the mammary tissue and/or cell sample, the mammary tissue and/or cell sample is determined as a healthy mammary tissue and/or cell sample; and
if the imprinted gene Z18, Z19, Z21, Z22, or Z27 is expressed as having a loss of imprinting and/or a copy number variation in the cell nuclei in the mammary tissue and/or cell sample, the mammary tissue and/or cell sample is determined as a mammary cancer tissue and/or cell sample;
or
(l) subjecting a thyroid tissue and/or cell sample to an analysis of the expression of the imprinted genes Z18, Z19, Z20, Z21, and Z23 so that:
if the imprinted genes Z18, Z19, Z20, Z21, and Z23 are not expressed or are expressed as normal in cell nuclei in the thyroid tissue and/or cell sample, the thyroid tissue and/or cell sample is determined as a healthy thyroid tissue and/or cell sample; and
if the imprinted gene Z18, Z19, Z20, Z21, or Z23 is expressed as having a loss of imprinting and/or a copy number variation in the cell nuclei in the thyroid tissue and/or cell sample, the thyroid tissue and/or cell sample is determined as a thyroid cancer tissue and/or cell sample;
or
(m) subjecting a bladder tissue and/or cell sample to an analysis of the expression of the imprinted genes Z18, Z19, Z20, Z21, Z22, Z26, Z27, and Z28 so that:
if the imprinted genes Z18, Z19, Z20, Z21, Z22, Z26, Z27, and Z28 are not expressed or are expressed as normal in cell nuclei in the bladder tissue and/or cell sample, the bladder tissue and/or cell sample is determined as a healthy bladder tissue and/or cell sample; and
if the imprinted gene Z18, Z19, Z20, Z21, Z22, Z26, Z27, or Z28 is expressed as having a loss of imprinting and/or a copy number variation in the cell nuclei in the bladder tissue and/or cell sample, the bladder tissue and/or cell sample is determined as a bladder cancer tissue and/or cell sample;
or
(n) subjecting a liver tissue and/or cell sample to an analysis of the expression of the imprinted genes Z17, Z19, Z21, Z22, Z26, Z27, and Z28 so that:
if the imprinted genes Z17, Z19, Z21, Z22, Z26, Z27, and Z28 are not expressed or are expressed as normal in cell nuclei in the liver tissue and/or cell sample, the liver tissue and/or cell sample is determined as a healthy liver tissue and/or cell sample; and
if the imprinted gene Z17, Z19, Z21, Z22, Z26, Z27, or Z28 is expressed as having a loss of imprinting and/or a copy number variation in the cell nuclei in the liver tissue and/or cell sample, the liver tissue and/or cell sample is determined as a liver cancer tissue and/or cell sample;
or
(o) subjecting a pancreatic tissue and/or cell sample to an analysis of the expression of the imprinted genes Z17, Z18, Z19, Z20, Z21, Z22, Z26, Z27, and Z28 so that:
if the imprinted genes Z17, Z18, Z19, Z20, Z21, Z22, Z26, Z27, and Z28 are not expressed or are expressed as normal in cell nuclei in the pancreatic tissue and/or cell sample, the pancreatic tissue and/or cell sample is determined as a healthy pancreatic tissue and/or cell sample; and
if the imprinted gene Z17, Z18, Z19, Z20, Z21, Z22, Z26, Z27, or Z28 is expressed as having a loss of imprinting and/or a copy number variation in the cell nuclei in the pancreatic tissue and/or cell sample, the pancreatic tissue and/or cell sample is determined as a pancreatic cancer tissue and/or cell sample;
or
(p) subjecting a lymphatic tissue and/or cell sample to an analysis of the expression of the imprinted genes Z18, Z19, Z21, Z22, Z26, Z27, and Z28 so that:
if the imprinted genes Z18, Z19, Z21, Z22, Z26, Z27, and Z28 are not expressed or are expressed as normal in cell nuclei in the lymphatic tissue and/or cell sample, the lymphatic tissue and/or cell sample is determined as a healthy lymphatic tissue and/or cell sample; and
if the imprinted gene Z18, Z19, Z21, Z22, Z26, Z27, or Z28 is expressed as having a loss of imprinting and/or a copy number variation in the cell nuclei in the lymphatic tissue and/or cell sample, the lymphatic tissue and/or cell sample is determined as a lymphoma tissue and/or cell sample;
or
(q) subjecting a colorectal tissue and/or cell sample to an analysis of the expression of the imprinted genes Z19, Z20, Z21, Z22, Z26, and Z27 so that:
if the imprinted genes Z19, Z20, Z21, Z22, Z26, and Z27 are not expressed or are expressed as normal in cell nuclei in the colorectal tissue and/or cell sample, the colorectal tissue and/or cell sample is determined as a healthy colorectal tissue and/or cell sample; and
if the imprinted gene Z19, Z20, Z21, Z22, Z26, or Z27 is expressed as having a loss of imprinting and/or a copy number variation in the cell nuclei in the colorectal tissue and/or cell sample, the colorectal tissue and/or cell sample is determined as a colorectal cancer tissue and/or cell sample;
or
(r) subjecting a skin tissue and/or cell sample to an analysis of the expression of the imprinted genes Z18, Z19, Z21, Z22, Z25, and Z28 so that:
if the imprinted genes Z18, Z19, Z21, Z22, Z25, and Z28 are not expressed or are expressed as normal in cell nuclei in the skin tissue and/or cell sample, the skin tissue and/or cell sample is determined as a healthy skin tissue and/or cell sample; and
if the imprinted gene Z18, Z19, Z21, Z22, Z25, or Z28 is expressed as having a loss of imprinting and/or a copy number variation in the cell nuclei in the skin tissue and/or cell sample, the skin tissue and/or cell sample is determined as a skin cancer tissue and/or cell sample;
or
(s) subjecting a lung tissue and/or cell sample to an analysis of the expression of the imprinted genes Z18, Z19, Z21, Z22, Z25, Z26, Z27, and Z28 so that:
if the imprinted genes Z18, Z19, Z21, Z22, Z25, Z26, Z27, and Z28 are not expressed or are expressed as normal in cell nuclei in the lung tissue and/or cell sample, the lung tissue and/or cell sample is determined as a healthy lung tissue and/or cell sample; and
if the imprinted gene Z18, Z19, Z21, Z22, Z25, Z26, Z27, or Z28 is expressed as having a loss of imprinting and/or a copy number variation in the cell nuclei in the lung tissue and/or cell sample, the lung tissue and/or cell sample is determined as a lung cancer tissue and/or cell sample;
wherein the imprinted gene Z1 is Gnas, the imprinted gene Z2 is Igf2, the imprinted gene Z3 is Peg10, the imprinted gene Z4 is Igf2r, the imprinted gene Z5 is Mest, the imprinted gene Z6 is Plagl1, the imprinted gene Z8 is Dcn, the imprinted gene Z9 is Dlk1, the imprinted gene Z10 is Gatm, the imprinted gene N11 is Grb10, the imprinted gene Z12 is Peg3, the imprinted gene Z13 is Sgce, the imprinted gene Z14 is Slc38a4, the imprinted gene Z15 is Diras3, the imprinted gene N16 is Snrpn/Snurf, the imprinted gene Z17 is Aldh1l1, the imprinted gene Z18 is Gata3, the imprinted gene Z19 is Hm13, the imprinted gene Z20 is Kenq1, the imprinted gene Z21 is Nhp2l1, the imprinted gene Z22 is Pon2, the imprinted gene Z23 is Rasgrf1, the imprinted gene Z25 is Tfpi2, the imprinted gene Z26 is Trappc9, the imprinted gene Z27 is Ube3a, and the imprinted gene Z28 is Zic1.

2. The method of claim 1, wherein the sample is sourced from tissue and/or cells.

3. The method of claim 2, wherein the in-situ hybridization uses an RNAscope in-situ hybridization method.

4. The method of claim 3, wherein the RNAscope in-situ hybridization method uses a single-or multiple-channel chromogenic reagent kit or a single- or multiple-channel fluorescent reagent kit.

5. The method of claim 4, wherein the RNAscope in-situ hybridization method uses a single-channel red/brown chromogenic reagent kit or a multiple-channel fluorescent reagent kit.

6. The method of claim 4 or claim 5, wherein:
a said imprinted gene is determined as not being expressed when no imprinted gene mark is present in the nucleus of a sample cell;
the imprinted gene is determined as being expressed as normal when only one imprinted gene mark is present in the nucleus of the sample cell;
the imprinted gene is determined as having a loss of imprinting when two imprinted gene marks are present in the nucleus of the sample cell; and
the imprinted gene is determined as having a copy number variation when more than two imprinted gene marks are present in the nucleus of the sample cell.

7. A tumor diagnosis device, comprising:
a sampling unit for obtaining a sample;
a probe designing unit for designing a probe specific to the sequence of an imprinted gene to be detected;
a detection unit for performing in-situ hybridization between the probe and the sample; and
an analysis unit for analyzing expression of the imprinted gene through microscopic imaging;
wherein the tumor diagnosis device is configured to carry out tumor diagnosis by using an analysis method comprising:
(a) subjecting a bile duct tissue and/or cell sample to an analysis of the expression of the imprinted genes Z1, Z2, Z8, Z10, N11, Z14, N16, Z19, Z20, Z21, Z22, Z25, Z26, and Z27 so that:
if the imprinted genes Z1, Z2, Z8, Z10, N11, Z14, N16, Z19, Z20, Z21, Z22, Z25, Z26, and Z27 are not expressed or are expressed as normal in cell nuclei in the bile duct tissue and/or cell sample, the bile duct tissue and/or cell sample is determined as a healthy bile duct tissue and/or cell sample; and
if the imprinted gene Z1, Z2, Z8, Z10, N11, Z14, N16, Z19, Z20, Z21, Z22, Z25, Z26, or Z27 is expressed as having a loss of imprinting and/or a copy number variation in the cell nuclei in the bile duct tissue and/or cell sample, the bile duct tissue and/or cell sample is determined as a cholangiocarcinoma tissue and/or cell sample;
or
(b) subjecting a brain tissue and/or cell sample to an analysis of the expression of the imprinted genes Z1, Z2, Z4, Z5, Z8, Z9, Z10, N11, Z13, Z14, N16, Z17, Z19, Z21, Z22, Z23, Z26, Z27, and Z28 so that:
if the imprinted genes Z1, Z2, Z4, Z5, Z8, Z9, Z10, N11, Z13, Z14, N16, Z17, Z19, Z21, Z22, Z23, Z26, Z27, and Z28 are not expressed or are expressed as normal in cell nuclei in the brain tissue and/or cell sample, the brain tissue and/or cell sample is determined as a healthy brain tissue and/or cell sample; and
if the imprinted gene Z1, Z2, Z4, Z5, Z8, Z9, Z10, N11, Z13, Z14, N16, Z17, Z19, Z21, Z22, Z23, Z26, Z27, or Z28 is expressed as having a loss of imprinting and/or a copy number variation in the cell nuclei in the brain tissue and/or cell sample, the brain tissue and/or cell sample is determined as a brain cancer tissue and/or cell sample;
or
(c) subjecting a kidney tissue and/or cell sample to an analysis of the expression of the imprinted genes Z1, Z4, Z6, Z8, Z10, N11, Z13, N16, Z17, Z18, Z19, Z20, Z21, Z25, Z26, Z27, and Z28 so that:
if the imprinted genes Z1, Z4, Z6, Z8, Z10, N11, Z13, N16, Z17, Z18, Z19, Z20, Z21, Z25, Z26, Z27, and Z28 are not expressed or are expressed as normal in cell nuclei in the kidney tissue and/or cell sample, the kidney tissue and/or cell sample is determined as a healthy kidney tissue and/or cell sample; and
if the imprinted gene Z1, Z4, Z6, Z8, Z10, N11, Z13, N16, Z17, Z18, Z19, Z20, Z21, Z25, Z26, Z27, or Z28 is expressed as having a loss of imprinting and/or a copy number variation in the cell nuclei in the kidney tissue and/or cell sample, the kidney tissue and/or cell sample is determined as a kidney cancer tissue and/or cell sample;
or
(d) subjecting a bone tissue and/or cell sample to an analysis of the expression of the imprinted genes Z1, Z2, Z3, Z6, Z8, Z9, Z10, N11, Z13, N16, Z18, Z19, Z20, Z21, Z22, Z25, Z26, Z27, and Z28 so that:
if the imprinted genes Z1, Z2, Z3, Z6, Z8, Z9, Z10, N11, Z13, N16, Z18, Z19, Z20, Z21, Z22, Z25, Z26, Z27, and Z28 are not expressed or are expressed as normal in cell nuclei in the bone tissue and/or cell sample, the bone tissue and/or cell sample is determined as a healthy bone tissue and/or cell sample; and
if the imprinted gene Z1, Z2, Z3, Z6, Z8, Z9, Z10, N11, Z13, N16, Z18, Z19, Z20, Z21, Z22, Z25, Z26, Z27, or Z28 is expressed as having a loss of imprinting and/or a copy number variation in the cell nuclei in the bone tissue and/or cell sample, the bone tissue and/or cell sample is determined as a bone cancer tissue and/or cell sample;
or
(e) subjecting a cervical tissue and/or cell sample to an analysis of the expression of the imprinted genes Z1, Z3, Z5, Z8, Z9, Z10, N11, N16, Z17, Z19, Z20, Z21, Z22, Z23, Z26, Z27, and Z28 so that:
if the imprinted genes Z1, Z3, Z5, Z8, Z9, Z10, N11, N16, Z17, Z19, Z20, Z21, Z22, Z23, Z26, Z27, and Z28 are not expressed or are expressed as normal in cell nuclei in the cervical tissue and/or cell sample, the cervical tissue and/or cell sample is determined as a healthy cervical tissue and/or cell sample; and
if the imprinted gene Z1, Z3, Z5, Z8, Z9, Z10, N11, N16, Z17, Z19, Z20, Z21, Z22, Z23, Z26, Z27, or Z28 is expressed as having a loss of imprinting and/or a copy number variation in the cell nuclei in the cervical tissue and/or cell sample, the cervical tissue and/or cell sample is determined as a cervical cancer tissue and/or cell sample;
or
(f) subjecting a nasopharyngeal tissue and/or cell sample to an analysis of the expression of the imprinted genes Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, and Z28 so that:
if the imprinted genes Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, and Z28 are not expressed or are expressed as normal in cell nuclei in the nasopharyngeal tissue and/or cell sample, the nasopharyngeal tissue and/or cell sample is determined as a healthy nasopharyngeal tissue and/or cell sample; and
if the imprinted gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27, or Z28 is expressed as having a loss of imprinting and/or a copy number variation in the cell nuclei in the nasopharyngeal tissue and/or cell sample, the nasopharyngeal tissue and/or cell sample is determined as a nasopharyngeal carcinoma tissue and/or cell sample;
or
(g) subjecting a laryngeal tissue and/or cell sample to an analysis of the expression of the imprinted genes Z1, Z3, Z6, Z8, N11, Z15, N16, Z17, Z18, Z19, Z21, Z22, Z25, Z26, Z27, and Z28 so that:
if the imprinted genes Z1, Z3, Z6, Z8, N11, Z15, N16, Z17, Z18, Z19, Z21, Z22, Z25, Z26, Z27, and Z28 are not expressed or are expressed as normal in cell nuclei in the laryngeal tissue and/or cell sample, the laryngeal tissue and/or cell sample is determined as a healthy laryngeal tissue and/or cell sample; and
if the imprinted gene Z1, Z3, Z6, Z8, N11, Z15, N16, Z17, Z18, Z19, Z21, Z22, Z25, Z26, Z27, or Z28 is expressed as having a loss of imprinting and/or a copy number variation in the cell nuclei in the laryngeal tissue and/or cell sample, the laryngeal tissue and/or cell sample is determined as a laryngeal cancer tissue and/or cell sample;
or
(h) subjecting a prostate tissue and/or cell sample to an analysis of the expression of of the imprinted genes Z18, Z19, Z20, Z21, Z25, Z26, Z27, and Z28 so that:
if the imprinted genes Z18, Z19, Z20, Z21, Z25, Z26, Z27, and Z28 are not expressed or are expressed as normal in cell nuclei in the prostate tissue and/or cell sample, the prostate tissue and/or cell sample is determined as a healthy prostate tissue and/or cell sample; and
if the imprinted gene Z18, Z19, Z20, Z21, Z25, Z26, Z27, or Z28 is expressed as having a loss of imprinting and/or a copy number variation in the cell nuclei in the prostate tissue and/or cell sample, the prostate tissue and/or cell sample is determined as a prostate cancer tissue and/or cell sample;
or
(i) subjecting a gastric tissue and/or cell sample to an analysis of the expression of the imprinted genes Z18, Z19, Z20, Z21, Z22, Z26, and Z27 so that:
if the imprinted genes Z18, Z19, Z20, Z21, Z22, Z26, and Z27 are not expressed or are expressed as normal in cell nuclei in the gastric tissue and/or cell sample, the gastric tissue and/or cell sample is determined as a healthy gastric tissue and/or cell sample; and
if the imprinted gene Z18, Z19, Z20, Z21, Z22, Z26, or Z27 is expressed as having a loss of imprinting and/or a copy number variation in the cell nuclei in the gastric tissue and/or cell sample, the gastric tissue and/or cell sample is determined as a gastric carcinoma tissue and/or cell sample;
or
(j) subjecting an esophageal tissue and/or cell sample to an analysis of the expression of the imprinted genes Z18, Z19, Z21, Z22, Z26, Z27, and Z28 so that:
if the imprinted genes Z18, Z19, Z21, Z22, Z26, Z27, and Z28 are not expressed or are expressed as normal in cell nuclei in the esophageal tissue and/or cell sample, the esophageal tissue and/or cell sample is determined as a healthy esophageal tissue and/or cell sample; and
if the imprinted gene Z18, Z19, Z21, Z22, Z26, Z27, or Z28 is expressed as having a loss of imprinting and/or a copy number variation in the cell nuclei in the esophageal tissue and/or cell sample, the esophageal tissue and/or cell sample is determined as an esophageal cancer tissue and/or cell sample;
or
(k) subjecting a mammary tissue and/or cell sample to an analysis of the expression of the imprinted genes Z18, Z19, Z21, Z22, and Z27 so that:
if the imprinted genes Z18, Z19, Z21, Z22, and Z27 are not expressed or are expressed as normal in cell nuclei in the mammary tissue and/or cell sample, the mammary tissue and/or cell sample is determined as a healthy mammary tissue and/or cell sample; and
if the imprinted gene Z18, Z19, Z21, Z22, or Z27 is expressed as having a loss of imprinting and/or a copy number variation in the cell nuclei in the mammary tissue and/or cell sample, the mammary tissue and/or cell sample is determined as a mammary cancer tissue and/or cell sample;
or
(l) subjecting a thyroid tissue and/or cell sample to an analysis of the expression of the imprinted genes Z18, Z19, Z20, Z21, and Z23 so that:
if the imprinted genes Z18, Z19, Z20, Z21, and Z23 are not expressed or are expressed as normal in cell nuclei in the thyroid tissue and/or cell sample, the thyroid tissue and/or cell sample is determined as a healthy thyroid tissue and/or cell sample; and
if the imprinted gene Z18, Z19, Z20, Z21, or Z23 is expressed as having a loss of imprinting and/or a copy number variation in the cell nuclei in the thyroid tissue and/or cell sample, the thyroid tissue and/or cell sample is determined as a thyroid cancer tissue and/or cell sample;
or
(m) subjecting a bladder tissue and/or cell sample to an analysis of the expression of the imprinted genes Z18, Z19, Z20, Z21, Z22, Z26, Z27, and Z28 so that:
if the imprinted genes Z18, Z19, Z20, Z21, Z22, Z26, Z27, and Z28 are not expressed or are expressed as normal in cell nuclei in the bladder tissue and/or cell sample, the bladder tissue and/or cell sample is determined as a healthy bladder tissue and/or cell sample; and
if the imprinted gene Z18, Z19, Z20, Z21, Z22, Z26, Z27, or Z28 is expressed as having a loss of imprinting and/or a copy number variation in the cell nuclei in the bladder tissue and/or cell sample, the bladder tissue and/or cell sample is determined as a bladder cancer tissue and/or cell sample;
or
(n) subjecting a liver tissue and/or cell sample to an analysis of the expression of the imprinted genes Z17, Z19, Z21, Z22, Z26, Z27, and Z28 so that:
if the imprinted genes Z17, Z19, Z21, Z22, Z26, Z27, and Z28 are not expressed or are expressed as normal in cell nuclei in the liver tissue and/or cell sample, the liver tissue and/or cell sample is determined as a healthy liver tissue and/or cell sample; and
if the imprinted gene Z17, Z19, Z21, Z22, Z26, Z27, or Z28 is expressed as having a loss of imprinting and/or a copy number variation in the cell nuclei in the liver tissue and/or cell sample, the liver tissue and/or cell sample is determined as a liver cancer tissue and/or cell sample;
or
(o) subjecting a pancreatic tissue and/or cell sample to an analysis of the expression of the imprinted genes Z17, Z18, Z19, Z20, Z21, Z22, Z26, Z27, and Z28 so that:
if the imprinted genes Z17, Z18, Z19, Z20, Z21, Z22, Z26, Z27, and Z28 are not expressed or are expressed as normal in cell nuclei in the pancreatic tissue and/or cell sample, the pancreatic tissue and/or cell sample is determined as a healthy pancreatic tissue and/or cell sample; and
if the imprinted gene Z17, Z18, Z19, Z20, Z21, Z22, Z26, Z27, or Z28 is expressed as having a loss of imprinting and/or a copy number variation in the cell nuclei in the pancreatic tissue and/or cell sample, the pancreatic tissue and/or cell sample is determined as a pancreatic cancer tissue and/or cell sample;
or
(p) subjecting a lymphatic tissue and/or cell sample to an analysis of the expression of the imprinted genes Z18, Z19, Z21, Z22, Z26, Z27, and Z28 so that:
if the imprinted genes Z18, Z19, Z21, Z22, Z26, Z27, and Z28 are not expressed or are expressed as normal in cell nuclei in the lymphatic tissue and/or cell sample, the lymphatic tissue and/or cell sample is determined as a healthy lymphatic tissue and/or cell sample; and
if the imprinted gene Z18, Z19, Z21, Z22, Z26, Z27, or Z28 is expressed as having a loss of imprinting and/or a copy number variation in the cell nuclei in the lymphatic tissue and/or cell sample, the lymphatic tissue and/or cell sample is determined as a lymphoma tissue and/or cell sample;
or
(q) subjecting a colorectal tissue and/or cell sample to an analysis of the expression of the imprinted genes Z19, Z20, Z21, Z22, Z26, and Z27 so that:
if the imprinted genes Z19, Z20, Z21, Z22, Z26, and Z27 are not expressed or are expressed as normal in cell nuclei in the colorectal tissue and/or cell sample, the colorectal tissue and/or cell sample is determined as a healthy colorectal tissue and/or cell sample; and
if the imprinted gene Z19, Z20, Z21, Z22, Z26, or Z27 is expressed as having a loss of imprinting and/or a copy number variation in the cell nuclei in the colorectal tissue and/or cell sample, the colorectal tissue and/or cell sample is determined as a colorectal cancer tissue and/or cell sample;
or
(r) subjecting a skin tissue and/or cell sample to an analysis of the expression of the imprinted genes Z18, Z19, Z21, Z22, Z25, and Z28 so that:
if the imprinted genes Z18, Z19, Z21, Z22, Z25, and Z28 are not expressed or are expressed as normal in cell nuclei in the skin tissue and/or cell sample, the skin tissue and/or cell sample is determined as a healthy skin tissue and/or cell sample; and
if the imprinted gene Z18, Z19, Z21, Z22, Z25, or Z28 is expressed as having a loss of imprinting and/or a copy number variation in the cell nuclei in the skin tissue and/or cell sample, the skin tissue and/or cell sample is determined as a skin cancer tissue and/or cell sample;
or
(s) subjecting a lung tissue and/or cell sample to an analysis of the expression of the imprinted genes Z18, Z19, Z21, Z22, Z25, Z26, Z27, and Z28 so that:
if the imprinted genes Z18, Z19, Z21, Z22, Z25, Z26, Z27, and Z28 are not expressed or are expressed as normal in cell nuclei in the lung tissue and/or cell sample, the lung tissue and/or cell sample is determined as a healthy lung tissue and/or cell sample; and
if the imprinted gene Z18, Z19, Z21, Z22, Z25, Z26, Z27, or Z28 is expressed as having a loss of imprinting and/or a copy number variation in the cell nuclei in the lung tissue and/or cell sample, the lung tissue and/or cell sample is determined as a lung cancer tissue and/or cell sample.

## Patentansprüche

1. Ein Verfahren zur Tumordiagnose umfassend:
(a) Unterziehen einer Gallengangsgewebe- und/oder -zellprobe einer Analyse der Expression der geprägten Gene Z1, Z2, Z8, Z10, N11, Z14, N16, Z19, Z20, Z21, Z22, Z25, Z26 und Z27, so dass:
falls die geprägten Gene Z1, Z2, Z8, Z10, N11, Z14, N16, Z19, Z20, Z21, Z22, Z25, Z26 und Z27 in der Gallengangsgewebe- und/oder -zellprobe in Zellkernen nicht exprimiert sind oder normal exprimiert sind, wird die Gallengangsgewebe- und/oder -zellprobe als eine gesunde Gallengangsgewebe- und/oder -zellprobe bestimmt; und
falls die geprägten Gene Z1, Z2, Z8, Z10, N11, Z14, N16, Z19, Z20, Z21, Z22, Z25, Z26 und Z27 in der Gallengangsgewebe- und/oder -zellprobe in Zellkernen mit einem Verlust der Prägung und/oder einer Kopienanzahlvariation exprimiert sind, wird die Gallengangsgewebe- und/oder -zellprobe als eine Cholangiokarzinomgewebe- und/oder -zellprobe bestimmt;
oder
(b) Unterziehen einer Hirngewebe- und/oder -zellprobe einer Analyse der Expression der geprägten Gene Z1, Z2, Z4, Z5, Z8, Z9, Z10, N11, Z13, Z14, N16, Z17, Z19, Z21, Z22, Z23, Z26, Z27 und Z28, so dass:
falls die geprägten Gene Z1, Z2, Z4, Z5, Z8, Z9, Z10, N11, Z13, Z14, N16, Z17, Z19, Z21, Z22, Z23, Z26, Z27 und Z28 in der Hirngewebe- und/oder -zellprobe in Zellkernen nicht exprimiert sind oder normal exprimiert sind, wird die Hirngewebe- und/oder -zellprobe als eine gesunde Hirngewebe- und/oder -zellprobe bestimmt; und
falls die geprägten Gene Z1, Z2, Z4, Z5, Z8, Z9, Z10, N11, Z13, Z14, N16, Z17, Z19, Z21, Z22, Z23, Z26, Z27 und Z28 in der Hirngewebe- und/oder -zellprobe in Zellkernen mit einem Verlust der Prägung und/oder einer Kopienanzahlvariation exprimiert sind, wird die Hirngewebe-und/oder -zellprobe als eine Hirnkrebsgewebe- und/oder -zellprobe bestimmt;
oder
(c) Unterziehen einer Nierengewebe- und/oder -zellprobe einer Analyse der Expression der geprägten Gene Z1, Z4, Z6, Z8, Z10, N11, Z13, N16, Z17, Z18, Z19, Z20, Z21, Z25, Z26, Z27 und Z28, so dass:
falls die geprägten Gene Z1, Z4, Z6, Z8, Z10, N11, Z13, N16, Z17, Z18, Z19, Z20, Z21, Z25, Z26, Z27 und Z28 in der Nierengewebe- und/oder -zellprobe in Zellkernen nicht exprimiert sind oder normal exprimiert sind, wird die Nierengewebe- und/oder -zellprobe als eine gesunde Nierengewebe- und/oder -zellprobe bestimmt; und
falls die geprägten Gene Z1, Z4, Z6, Z8, Z10, N11, Z13, N16, Z17, Z18, Z19, Z20, Z21, Z25, Z26, Z27 und Z28 in der Nierengewebe- und/oder -zellprobe in Zellkernen mit einem Verlust der Prägung und/oder einer Kopienanzahlvariation exprimiert sind, wird die Nierengewebe- und/oder -zellprobe als eine Nierenkrebsgewebe- und/oder -zellprobe bestimmt;
oder
(d) Unterziehen einer Knochengewebe- und/oder -zellprobe einer Analyse der Expression der geprägten Gene Z1, Z2, Z3, Z6, Z8, Z9, Z10, N11, Z13, N16, Z18, Z19, Z20, Z21, Z22, Z25, Z26, Z27 und Z28, so dass:
falls die geprägten Gene Z1, Z2, Z3, Z6, Z8, Z9, Z10, N11, Z13, N16, Z18, Z19, Z20, Z21, Z22, Z25, Z26, Z27 und Z28 in der Knochengewebe- und/oder -zellprobe in Zellkernen nicht exprimiert sind oder normal exprimiert sind, wird die Knochengewebe- und/oder -zellprobe als eine gesunde Knochengewebe- und/oder -zellprobe bestimmt; und
falls die geprägten Gene Z1, Z2, Z3, Z6, Z8, Z9, Z10, N11, Z13, N16, Z18, Z19, Z20, Z21, Z22, Z25, Z26, Z27 und Z28 in der Knochengewebe- und/oder -zellprobe in Zellkernen mit einem Verlust der Prägung und/oder einer Kopienanzahlvariation exprimiert sind, wird die Knochengewebe- und/oder -zellprobe als eine Knochenkrebsgewebe- und/oder -zellprobe bestimmt;
oder
(e) Unterziehen einer Gebärmutterhalsgewebe- und/oder -zellprobe einer Analyse der Expression der geprägten Gene Z1, Z3, Z5, Z8, Z9, Z10, N11, N16, Z17, Z19, Z20, Z21, Z22, Z23, Z26, Z27 und Z28, so dass:
falls die geprägten Gene Z1, Z3, Z5, Z8, Z9, Z10, N11, N16, Z17, Z19, Z20, Z21, Z22, Z23, Z26, Z27 und Z28 in der Gebärmutterhalsgewebe- und/oder -zellprobe in Zellkernen nicht exprimiert sind oder normal exprimiert sind, wird die Gebärmutterhalsgewebe- und/oder -zellprobe als eine gesunde Gebärmutterhalsgewebe- und/oder -zellprobe bestimmt; und
falls die geprägten Gene Z1, Z3, Z5, Z8, Z9, Z10, N11, N16, Z17, Z19, Z20, Z21, Z22, Z23, Z26, Z27 und Z28 in der Gebärmutterhalsgewebe- und/oder -zellprobe in Zellkernen mit einem Verlust der Prägung und/oder einer Kopienanzahlvariation exprimiert sind, wird die Gebärmutterhalsgewebe- und/oder -zellprobe als eine Gebärmutterhalskrebsgewebe- und/oder -zellprobe bestimmt;
oder
(f) Unterziehen einer nasopharyngealen Gewebe- und/oder -zellprobe einer Analyse der Expression der geprägten Gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 und Z28, so dass:
falls die geprägten Gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 und Z28 in der nasopharyngealen Gewebe- und/oder -zellprobe in Zellkernen nicht exprimiert sind oder normal exprimiert sind, wird die nasopharyngeale Gewebe- und/oder -zellprobe als eine gesunde nasopharyngeale Gewebe-und/oder -zellprobe bestimmt; und
falls die geprägten Gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 und Z28 in der nasopharyngealen Gewebe- und/oder -zellprobe in Zellkernen mit einem Verlust der Prägung und/oder einer Kopienanzahlvariation exprimiert sind, wird die nasopharyngeale Gewebe- und/oder -zellprobe als eine nasopharyngeale Karzinomgewebe- und/oder -zellprobe bestimmt;
oder
(g) Unterziehen einer Kehlkopfgewebe- und/oder -zellprobe einer Analyse der Expression der geprägten Gene Z1, Z3, Z6, Z8, N11, Z15, N16, Z17, Z18, Z19, Z21, Z22, Z25, Z26, Z27 und Z28, so dass:
falls die geprägten Gene Z1, Z3, Z6, Z8, N11, Z15, N16, Z17, Z18, Z19, Z21, Z22, Z25, Z26, Z27 und Z28 in der Kehlkopfgewebe- und/oder -zellprobe in Zellkernen nicht exprimiert sind oder normal exprimiert sind, wird die Kehlkopfgewebe- und/oder -zellprobe als eine gesunde Kehlkopfgewebe- und/oder -zellprobe bestimmt; und
falls die geprägten Gene Z1, Z3, Z6, Z8, N11, Z15, N16, Z17, Z18, Z19, Z21, Z22, Z25, Z26, Z27 und Z28 in der Kehlkopfgewebe- und/oder -zellprobe in Zellkernen mit einem Verlust der Prägung und/oder einer Kopienanzahlvariation exprimiert sind, wird die Kehlkopfgewebe-und/oder -zellprobe als eine Kehlkopfkrebsgewebe- und/oder -zellprobe bestimmt;
oder
(h) Unterziehen einer Prostatagewebe- und/oder -zellprobe einer Analyse der Expression der geprägten Gene Z18, Z19, Z20, Z21, Z25, Z26, Z27 und Z28, so dass:
falls die geprägten Gene Z18, Z19, Z20, Z21, Z25, Z26, Z27 und Z28 in der Prostatagewebe-und/oder -zellprobe in Zellkernen nicht exprimiert sind oder normal exprimiert sind, wird die Prostatagewebe- und/oder -zellprobe als eine gesunde Prostatagewebe- und/oder -zellprobe bestimmt; und
falls die geprägten Gene Z18, Z19, Z20, Z21, Z25, Z26, Z27 und Z28 in der Prostatagewebe-und/oder -zellprobe in Zellkernen mit einem Verlust der Prägung und/oder einer Kopienanzahlvariation exprimiert sind, wird die Prostatagewebe- und/oder -zellprobe als eine Prostatakrebsgewebe- und/oder -zellprobe bestimmt;
oder
(i) Unterziehen einer Magengewebe- und/oder -zellprobe einer Analyse der Expression der geprägten Gene Z18, Z19, Z20, Z21, Z22, Z26 und Z27, so dass:
falls die geprägten Gene Z18, Z19, Z20, Z21, Z22, Z26 und Z27 in der Magengewebe-und/oder -zellprobe in Zellkernen nicht exprimiert sind oder normal exprimiert sind, wird die Magengewebe- und/oder -zellprobe als eine gesunde Magengewebe- und/oder -zellprobe bestimmt; und
falls die geprägten Gene Z18, Z19, Z20, Z21, Z22, Z26 und Z27 in der Magengewebe-und/oder -zellprobe in Zellkernen mit einem Verlust der Prägung und/oder einer Kopienanzahlvariation exprimiert sind, wird die Magengewebe- und/oder -zellprobe als eine Magenkarzinomgewebe- und/oder -zellprobe bestimmt;
oder
(j) Unterziehen einer Speiseröhrengewebe- und/oder -zellprobe einer Analyse der Expression der geprägten Gene Z18, Z19, Z21, Z22, Z26, Z27 und Z28, so dass:
falls die geprägten Gene Z18, Z19, Z21, Z22, Z26, Z27 und Z28 in der Speiseröhrengewebe-und/oder -zellprobe in Zellkernen nicht exprimiert sind oder normal exprimiert sind, wird die Speiseröhrengewebe- und/oder -zellprobe als eine gesunde Speiseröhrengewebe- und/oder -zellprobe bestimmt; und
falls die geprägten Gene Z18, Z19, Z21, Z22, Z26, Z27 und Z28 in der Speiseröhrengewebe-und/oder -zellprobe in Zellkernen mit einem Verlust der Prägung und/oder einer Kopienanzahlvariation exprimiert sind, wird die Speiseröhrengewebe- und/oder -zellprobe als eine Speiseröhrenkrebsgewebe- und/oder -zellprobe bestimmt;
oder
(k) Unterziehen einer Brustgewebe- und/oder -zellprobe einer Analyse der Expression der geprägten Gene Z18, Z19, Z21, Z22 und Z27, so dass:
falls die geprägten Gene Z18, Z19, Z21, Z22 und Z27 in der Brustgewebe- und/oder -zellprobe in Zellkernen nicht exprimiert sind oder normal exprimiert sind, wird die Brustgewebe-und/oder -zellprobe als eine gesunde Brustgewebe- und/oder -zellprobe bestimmt; und
falls die geprägten Gene Z18, Z19, Z21, Z22 und Z27 in der Brustgewebe- und/oder -zellprobe in Zellkernen mit einem Verlust der Prägung und/oder einer Kopienanzahlvariation exprimiert sind, wird die Brustgewebe- und/oder -zellprobe als eine Brustkrebsgewebe- und/oder -zellprobe bestimmt;
oder
(l) Unterziehen einer Schilddrüsengewebe- und/oder -zellprobe einer Analyse der Expression der geprägten Gene Z18, Z19, Z20, Z21 und Z23, so dass:
falls die geprägten Gene Z18, Z19, Z20, Z21 und Z23 in der Schilddrüsengewebe- und/oder -zellprobe in Zellkernen nicht exprimiert sind oder normal exprimiert sind, wird die Schilddrüsengewebe- und/oder -zellprobe als eine gesunde Schilddrüsengewebe- und/oder -zellprobe bestimmt; und
falls die geprägten Gene Z18, Z19, Z20, Z21 und Z23 in der Schilddrüsengewebe- und/oder -zellprobe in Zellkernen mit einem Verlust der Prägung und/oder einer Kopienanzahlvariation exprimiert sind, wird die Schilddrüsengewebe- und/oder -zellprobe als eine Schilddrüsenkrebsgewebe- und/oder -zellprobe bestimmt;
oder
(m) Unterziehen einer Blasengewebe- und/oder -zellprobe einer Analyse der Expression der geprägten Gene Z18, Z19, Z20, Z21, Z22, Z26, Z27 und Z28, so dass:
falls die geprägten Gene Z18, Z19, Z20, Z21, Z22, Z26, Z27 und Z28 in der Blasengewebe-und/oder -zellprobe in Zellkernen nicht exprimiert sind oder normal exprimiert sind, wird die Blasengewebe- und/oder -zellprobe als eine gesunde Blasengewebe- und/oder -zellprobe bestimmt; und
falls die geprägten Gene Z18, Z19, Z20, Z21, Z22, Z26, Z27 und Z28 in der Blasengewebe-und/oder -zellprobe in Zellkernen mit einem Verlust der Prägung und/oder einer Kopienanzahlvariation exprimiert sind, wird die Blasengewebe- und/oder -zellprobe als eine Blasenkrebsgewebe- und/oder -zellprobe bestimmt;
oder
(n) Unterziehen einer Lebergewebe- und/oder -zellprobe einer Analyse der Expression der geprägten Gene Z17, Z19, Z21, Z22, Z26, Z27 und Z28, so dass:
falls die geprägten Gene Z17, Z19, Z21, Z22, Z26, Z27 und Z28 in der Lebergewebe-und/oder -zellprobe in Zellkernen nicht exprimiert sind oder normal exprimiert sind, wird die Lebergewebe- und/oder -zellprobe als eine gesunde Lebergewebe- und/oder -zellprobe bestimmt; und
falls die geprägten Gene Z17, Z19, Z21, Z22, Z26, Z27 und Z28 in der Lebergewebe-und/oder -zellprobe in Zellkernen mit einem Verlust der Prägung und/oder einer Kopienanzahlvariation exprimiert sind, wird die Lebergewebe- und/oder -zellprobe als eine Leberkrebsgewebe- und/oder -zellprobe bestimmt;
oder
(o) Unterziehen einer Bauchspeicheldrüsengewebe- und/oder -zellprobe einer Analyse der Expression der geprägten Gene Z17, Z18, Z19, Z20, Z21, Z22, Z26, Z27 und Z28, so dass:
falls die geprägten Gene Z17, Z18, Z19, Z20, Z21, Z22, Z26, Z27 und Z28 in der Bauchspeicheldrüsengewebe- und/oder -zellprobe in Zellkernen nicht exprimiert sind oder normal exprimiert sind, wird die Bauchspeicheldrüsengewebe- und/oder -zellprobe als eine gesunde Bauchspeicheldrüsengewebe- und/oder -zellprobe bestimmt; und
falls die geprägten Gene Z17, Z18, Z19, Z20, Z21, Z22, Z26, Z27 und Z28 in der Lebergewebe- und/oder -zellprobe in Zellkernen mit einem Verlust der Prägung und/oder einer Kopienanzahlvariation exprimiert sind, wird die Bauchspeicheldrüsengewebe- und/oder -zellprobe als eine Bauchspeicheldrüsenkrebsgewebe- und/oder -zellprobe bestimmt;
oder
(p) Unterziehen einer lymphatischen Gewebe- und/oder -zellprobe einer Analyse der Expression der geprägten Gene Z18, Z19, Z21, Z22, Z26, Z27 und Z28, so dass:
falls die geprägten Gene Z18, Z19, Z21, Z22, Z26, Z27 und Z28 in der lymphatischen Gewebe- und/oder -zellprobe in Zellkernen nicht exprimiert sind oder normal exprimiert sind, wird die lymphatische Gewebe- und/oder -zellprobe als eine gesunde lymphatische Gewebe- und/oder -zellprobe bestimmt; und
falls die geprägten Gene Z18, Z19, Z21, Z22, Z26, Z27 und Z28 in der lymphatischen Gewebe- und/oder -zellprobe in Zellkernen mit einem Verlust der Prägung und/oder einer Kopienanzahlvariation exprimiert sind, wird die lymphatische Gewebe- und/oder -zellprobe als eine Lymphomgewebe- und/oder -zellprobe bestimmt;
oder
(q) Unterziehen einer kolorektalen Gewebe- und/oder -zellprobe einer Analyse der Expression der geprägten Gene Z19, Z20, Z21, Z22, Z26 und Z27, so dass:
falls die geprägten Gene Z19, Z20, Z21, Z22, Z26 und Z27in der kolorektalen Gewebe-und/oder -zellprobe in Zellkernen nicht exprimiert sind oder normal exprimiert sind, wird die kolorektale Gewebe- und/oder -zellprobe als eine gesunde kolorektale Gewebe- und/oder -zellprobe bestimmt; und
falls die geprägten Gene Z19, Z20, Z21, Z22, Z26 und Z27in der kolorektalen Gewebe-und/oder -zellprobe in Zellkernen mit einem Verlust der Prägung und/oder einer Kopienanzahlvariation exprimiert sind, wird die kolorektale Gewebe- und/oder -zellprobe als eine Darmkrebsgewebe- und/oder -zellprobe bestimmt;
oder
(r) Unterziehen einer Hautgewebe- und/oder -zellprobe einer Analyse der Expression der geprägten Gene Z18, Z19, Z21, Z22, Z25 und Z28, so dass:
falls die geprägten Gene Z18, Z19, Z21, Z22, Z25 und Z28 in der Hautgewebe- und/oder -zellprobe in Zellkernen nicht exprimiert sind oder normal exprimiert sind, wird die Hautgewebe-und/oder -zellprobe als eine gesunde Hautgewebe- und/oder -zellprobe bestimmt; und
falls die geprägten Gene Z18, Z19, Z21, Z22, Z25 und Z28 in der Hautgewebe- und/oder -zellprobe in Zellkernen mit einem Verlust der Prägung und/oder einer Kopienanzahlvariation exprimiert sind, wird die Hautgewebe- und/oder -zellprobe als eine Hautkrebsgewebe- und/oder -zellprobe bestimmt;
oder
(s) Unterziehen einer Lungengewebe- und/oder -zellprobe einer Analyse der Expression der geprägten Gene Z18, Z19, Z21, Z22, Z25, Z26, Z27 und Z28, so dass:
falls die geprägten Gene Z18, Z19, Z21, Z22, Z25, Z26, Z27 und Z28 in der Lungengewebe-und/oder -zellprobe in Zellkernen nicht exprimiert sind oder normal exprimiert sind, wird die Lungengewebe- und/oder -zellprobe als eine gesunde Lungengewebe- und/oder -zellprobe bestimmt; und
falls die geprägten Gene Z18, Z19, Z21, Z22, Z25, Z26, Z27 und Z28 in der Lungengewebe-und/oder -zellprobe in Zellkernen mit einem Verlust der Prägung und/oder einer Kopienanzahlvariation exprimiert sind, wird die Lungengewebe- und/oder -zellprobe als eine Lungenkrebsgewebe- und/oder -zellprobe bestimmt;
wobei das geprägte Gen Z1 Gnas ist, das geprägte Gen Z2 Igf2 ist, das geprägte Gen Z3 Peg10 ist, das geprägte Gen Z4 Igfr2 ist, das geprägte Gen Z5 Mest ist, das geprägte Gen Z6 Plagl1 ist, das geprägte Gen Z8 Dcn ist, das geprägte Gen Z9 Dlk1 ist, das geprägte Gen Z10 Gatm ist, das geprägte Gen N11 Grb10 ist, das geprägte Gen Z12 Peg3 ist, das geprägte Gen Z13 Sgce ist, das geprägte Gen Z14 Slc38a4 ist, das geprägte Gen Z15 Diras3 ist, das geprägte Gen N16 Snrpn/Snurf ist, das geprägte Gen Z17 Aldh1l1 ist, das geprägte Gen Z18 Gata3 ist, das geprägte Gen Z19 Hm13 ist, das geprägte Gen Z20 Kcnq1 ist, das geprägte Gen Z21 Nhp2l1 ist, das geprägte Gen Z22 Pon2 ist, das geprägte Gen Z23 Rasgrf1 ist, das geprägte Gen Z25 Tfpi2 ist, das geprägte Gen Z26 Trappc9 ist, das geprägte Gen Z27 Ube3a ist, und das geprägte Gen Z28 Zic1 ist.

2. Das Verfahren aus Anspruch 1, wobei die Probe aus Gewebe und/oder Zellenb stammt.

3. Das Verfahren aus Anspruch 2, wobei die in-situ-Hybridisierung ein RNAscope in-situ-Hybridisierungsverfahren verwendet.

4. Das Verfahren aus Anspruch 3, wobei das RNAscope in-situ-Hybridisierungsverfahren ein ein- oder mehrkanaliges chromogenes Reagenskit oder ein ein- oder mehrkanaliges fluoreszierendes Reagenskit verwendet.

5. Das Verfahren aus Anspruch 4, wobei das RNAscope in-situ-Hybridisierungsverfahren ein einkanaliges rot/braunes chromogenes Reagenskit oder ein mehrkanaliges fluoreszierendes Reagenskit verwendet.

6. Das Verfahren aus Anspruch 4 oder 5, wobei:
ein gegebenes geprägtes Gen als nicht exprimiert bestimmt wird, wenn keine Markierung des geprägten Gens im Kern einer Probenzelle vorliegt;
das geprägte Gen als normal exprimiert bestimmt wird, wenn nur eine Markierung des geprägten Gens im Kern der Probenzelle vorliegt;
das geprägte Gen als einen Verlust der Prägung habend bestimmt wird, wenn zwei Markierungen des geprägten Gens im Kern der Probenzelle vorliegen; und
das geprägte Gen als eine Kopienanzahlvariation habend bestimmt wird, wenn mehr als zwei Markierungen des geprägten Gens im Kern der Probenzelle vorliegen

7. Ein Tumordiagnosegerät, umfassend:
eine Probennahmeeinheit zum Erhalten einer Probe;
eine Sondenentwurfeinheit zum Entwerfen einer Sonde, die für die Sequenz eines nachzuweisenden geprägten Gens spezifisch ist;
eine Detektionseinheit zur Durchführung von in-situ-Hybridisierung zwischen der Sonde und der Probe; und
eine Analyseeinheit zum Analysieren der Expression des geprägten Gens durch mikroskopische Bildgebung;
wobei das Tumordiagnosegerät konfiguriert ist, Tumordiagnose durchzuführen mittels eines Analyseverfahrens umfassend:
(a) Unterziehen einer Gallengangsgewebe- und/oder -zellprobe einer Analyse der Expression der geprägten Gene Z1, Z2, Z8, Z10, N11, Z14, N16, Z19, Z20, Z21, Z22, Z25, Z26 und Z27, so dass:
falls die geprägten Gene Z1, Z2, Z8, Z10, N11, Z14, N16, Z19, Z20, Z21, Z22, Z25, Z26 und Z27 in der Gallengangsgewebe- und/oder -zellprobe in Zellkernen nicht exprimiert sind oder normal exprimiert sind, wird die Gallengangsgewebe- und/oder -zellprobe als eine gesunde Gallengangsgewebe- und/oder -zellprobe bestimmt; und
falls die geprägten Gene Z1, Z2, Z8, Z10, N11, Z14, N16, Z19, Z20, Z21, Z22, Z25, Z26 und Z27 in der Gallengangsgewebe- und/oder -zellprobe in Zellkernen mit einem Verlust der Prägung und/oder einer Kopienanzahlvariation exprimiert sind, wird die Gallengangsgewebe- und/oder -zellprobe als eine Cholangiokarzinomgewebe- und/oder -zellprobe bestimmt;
oder
(b) Unterziehen einer Hirngewebe- und/oder -zellprobe einer Analyse der Expression der geprägten Gene Z1, Z2, Z4, Z5, Z8, Z9, Z10, N11, Z13, Z14, N16, Z17, Z19, Z21, Z22, Z23, Z26, Z27 und Z28, so dass:
falls die geprägten Gene Z1, Z2, Z4, Z5, Z8, Z9, Z10, N11, Z13, Z14, N16, Z17, Z19, Z21, Z22, Z23, Z26, Z27 und Z28 in der Hirngewebe- und/oder -zellprobe in Zellkernen nicht exprimiert sind oder normal exprimiert sind, wird die Hirngewebe- und/oder -zellprobe als eine gesunde Hirngewebe- und/oder -zellprobe bestimmt; und
falls die geprägten Gene Z1, Z2, Z4, Z5, Z8, Z9, Z10, N11, Z13, Z14, N16, Z17, Z19, Z21, Z22, Z23, Z26, Z27 und Z28 in der Hirngewebe- und/oder -zellprobe in Zellkernen mit einem Verlust der Prägung und/oder einer Kopienanzahlvariation exprimiert sind, wird die Hirngewebe-und/oder -zellprobe als eine Hirnkrebsgewebe- und/oder -zellprobe bestimmt;
oder
(c) Unterziehen einer Nierengewebe- und/oder -zellprobe einer Analyse der Expression der geprägten Gene Z1, Z4, Z6, Z8, Z10, N11, Z13, N16, Z17, Z18, Z19, Z20, Z21, Z25, Z26, Z27 und Z28, so dass:
falls die geprägten Gene Z1, Z4, Z6, Z8, Z10, N11, Z13, N16, Z17, Z18, Z19, Z20, Z21, Z25, Z26, Z27 und Z28 in der Nierengewebe- und/oder -zellprobe in Zellkernen nicht exprimiert sind oder normal exprimiert sind, wird die Nierengewebe- und/oder -zellprobe als eine gesunde Nierengewebe- und/oder -zellprobe bestimmt; und
falls die geprägten Gene Z1, Z4, Z6, Z8, Z10, N11, Z13, N16, Z17, Z18, Z19, Z20, Z21, Z25, Z26, Z27 und Z28 in der Nierengewebe- und/oder -zellprobe in Zellkernen mit einem Verlust der Prägung und/oder einer Kopienanzahlvariation exprimiert sind, wird die Nierengewebe- und/oder -zellprobe als eine Nierenkrebsgewebe- und/oder -zellprobe bestimmt;
oder
(d) Unterziehen einer Knochengewebe- und/oder -zellprobe einer Analyse der Expression der geprägten Gene Z1, Z2, Z3, Z6, Z8, Z9, Z10, N11, Z13, N16, Z18, Z19, Z20, Z21, Z22, Z25, Z26, Z27 und Z28, so dass:
falls die geprägten Gene Z1, Z2, Z3, Z6, Z8, Z9, Z10, N11, Z13, N16, Z18, Z19, Z20, Z21, Z22, Z25, Z26, Z27 und Z28 in der Knochengewebe- und/oder -zellprobe in Zellkernen nicht exprimiert sind oder normal exprimiert sind, wird die Knochengewebe- und/oder -zellprobe als eine gesunde Knochengewebe- und/oder -zellprobe bestimmt; und
falls die geprägten Gene Z1, Z2, Z3, Z6, Z8, Z9, Z10, N11, Z13, N16, Z18, Z19, Z20, Z21, Z22, Z25, Z26, Z27 und Z28 in der Knochengewebe- und/oder -zellprobe in Zellkernen mit einem Verlust der Prägung und/oder einer Kopienanzahlvariation exprimiert sind, wird die Knochengewebe- und/oder -zellprobe als eine Knochenkrebsgewebe- und/oder -zellprobe bestimmt;
oder
(e) Unterziehen einer Gebärmutterhalsgewebe- und/oder -zellprobe einer Analyse der Expression der geprägten Gene Z1, Z3, Z5, Z8, Z9, Z10, N11, N16, Z17, Z19, Z20, Z21, Z22, Z23, Z26, Z27 und Z28, so dass:
falls die geprägten Gene Z1, Z3, Z5, Z8, Z9, Z10, N11, N16, Z17, Z19, Z20, Z21, Z22, Z23, Z26, Z27 und Z28 in der Gebärmutterhalsgewebe- und/oder -zellprobe in Zellkernen nicht exprimiert sind oder normal exprimiert sind, wird die Gebärmutterhalsgewebe- und/oder -zellprobe als eine gesunde Gebärmutterhalsgewebe- und/oder -zellprobe bestimmt; und
falls die geprägten Gene Z1, Z3, Z5, Z8, Z9, Z10, N11, N16, Z17, Z19, Z20, Z21, Z22, Z23, Z26, Z27 und Z28 in der Gebärmutterhalsgewebe- und/oder -zellprobe in Zellkernen mit einem Verlust der Prägung und/oder einer Kopienanzahlvariation exprimiert sind, wird die Gebärmutterhalsgewebe- und/oder -zellprobe als eine Gebärmutterhalskrebsgewebe- und/oder -zellprobe bestimmt;
oder
(f) Unterziehen einer nasopharyngealen Gewebe- und/oder -zellprobe einer Analyse der Expression der geprägten Gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 und Z28, so dass:
falls die geprägten Gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 und Z28 in der nasopharyngealen Gewebe- und/oder -zellprobe in Zellkernen nicht exprimiert sind oder normal exprimiert sind, wird die nasopharyngeale Gewebe- und/oder -zellprobe als eine gesunde nasopharyngeale Gewebe-und/oder -zellprobe bestimmt; und
falls die geprägten Gene Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 und Z28 in der nasopharyngealen Gewebe- und/oder -zellprobe in Zellkernen mit einem Verlust der Prägung und/oder einer Kopienanzahlvariation exprimiert sind, wird die nasopharyngeale Gewebe- und/oder -zellprobe als eine nasopharyngeale Karzinomgewebe- und/oder -zellprobe bestimmt;
oder
(g) Unterziehen einer Kehlkopfgewebe- und/oder -zellprobe einer Analyse der Expression der geprägten Gene Z1, Z3, Z6, Z8, N11, Z15, N16, Z17, Z18, Z19, Z21, Z22, Z25, Z26, Z27 und Z28, so dass:
falls die geprägten Gene Z1, Z3, Z6, Z8, N11, Z15, N16, Z17, Z18, Z19, Z21, Z22, Z25, Z26, Z27 und Z28 in der Kehlkopfgewebe- und/oder -zellprobe in Zellkernen nicht exprimiert sind oder normal exprimiert sind, wird die Kehlkopfgewebe- und/oder -zellprobe als eine gesunde Kehlkopfgewebe- und/oder -zellprobe bestimmt; und
falls die geprägten Gene Z1, Z3, Z6, Z8, N11, Z15, N16, Z17, Z18, Z19, Z21, Z22, Z25, Z26, Z27 und Z28 in der Kehlkopfgewebe- und/oder -zellprobe in Zellkernen mit einem Verlust der Prägung und/oder einer Kopienanzahlvariation exprimiert sind, wird die Kehlkopfgewebe-und/oder -zellprobe als eine Kehlkopfkrebsgewebe- und/oder -zellprobe bestimmt;
oder
(h) Unterziehen einer Prostatagewebe- und/oder -zellprobe einer Analyse der Expression der geprägten Gene Z18, Z19, Z20, Z21, Z25, Z26, Z27 und Z28, so dass:
falls die geprägten Gene Z18, Z19, Z20, Z21, Z25, Z26, Z27 und Z28 in der Prostatagewebe-und/oder -zellprobe in Zellkernen nicht exprimiert sind oder normal exprimiert sind, wird die Prostatagewebe- und/oder -zellprobe als eine gesunde Prostatagewebe- und/oder -zellprobe bestimmt; und
falls die geprägten Gene Z18, Z19, Z20, Z21, Z25, Z26, Z27 und Z28 in der Prostatagewebe-und/oder -zellprobe in Zellkernen mit einem Verlust der Prägung und/oder einer Kopienanzahlvariation exprimiert sind, wird die Prostatagewebe- und/oder -zellprobe als eine Prostatakrebsgewebe- und/oder -zellprobe bestimmt;
oder
(i) Unterziehen einer Magengewebe- und/oder -zellprobe einer Analyse der Expression der geprägten Gene Z18, Z19, Z20, Z21, Z22, Z26 und Z27, so dass:
falls die geprägten Gene Z18, Z19, Z20, Z21, Z22, Z26 und Z27 in der Magengewebe-und/oder -zellprobe in Zellkernen nicht exprimiert sind oder normal exprimiert sind, wird die Magengewebe- und/oder -zellprobe als eine gesunde Magengewebe- und/oder -zellprobe bestimmt; und
falls die geprägten Gene Z18, Z19, Z20, Z21, Z22, Z26 und Z27 in der Magengewebe-und/oder -zellprobe in Zellkernen mit einem Verlust der Prägung und/oder einer Kopienanzahlvariation exprimiert sind, wird die Magengewebe- und/oder -zellprobe als eine Magenkarzinomgewebe- und/oder -zellprobe bestimmt;
oder
(j) Unterziehen einer Speiseröhrengewebe- und/oder -zellprobe einer Analyse der Expression der geprägten Gene Z18, Z19, Z21, Z22, Z26, Z27 und Z28, so dass:
falls die geprägten Gene Z18, Z19, Z21, Z22, Z26, Z27 und Z28 in der Speiseröhrengewebe-und/oder -zellprobe in Zellkernen nicht exprimiert sind oder normal exprimiert sind, wird die Speiseröhrengewebe- und/oder -zellprobe als eine gesunde Speiseröhrengewebe- und/oder -zellprobe bestimmt; und
falls die geprägten Gene Z18, Z19, Z21, Z22, Z26, Z27 und Z28 in der Speiseröhrengewebe-und/oder -zellprobe in Zellkernen mit einem Verlust der Prägung und/oder einer Kopienanzahlvariation exprimiert sind, wird die Speiseröhrengewebe- und/oder -zellprobe als eine Speiseröhrenkrebsgewebe- und/oder -zellprobe bestimmt;
oder
(k) Unterziehen einer Brustgewebe- und/oder -zellprobe einer Analyse der Expression der geprägten Gene Z18, Z19, Z21, Z22 und Z27, so dass:
falls die geprägten Gene Z18, Z19, Z21, Z22 und Z27 in der Brustgewebe- und/oder -zellprobe in Zellkernen nicht exprimiert sind oder normal exprimiert sind, wird die Brustgewebe-und/oder -zellprobe als eine gesunde Brustgewebe- und/oder -zellprobe bestimmt; und
falls die geprägten Gene Z18, Z19, Z21, Z22 und Z27 in der Brustgewebe- und/oder -zellprobe in Zellkernen mit einem Verlust der Prägung und/oder einer Kopienanzahlvariation exprimiert sind, wird die Brustgewebe- und/oder -zellprobe als eine Brustkrebsgewebe- und/oder -zellprobe bestimmt;
oder
(l) Unterziehen einer Schilddrüsengewebe- und/oder -zellprobe einer Analyse der Expression der geprägten Gene Z18, Z19, Z20, Z21 und Z23, so dass:
falls die geprägten Gene Z18, Z19, Z20, Z21 und Z23 in der Schilddrüsengewebe- und/oder -zellprobe in Zellkernen nicht exprimiert sind oder normal exprimiert sind, wird die Schilddrüsengewebe- und/oder -zellprobe als eine gesunde Schilddrüsengewebe- und/oder -zellprobe bestimmt; und
falls die geprägten Gene Z18, Z19, Z20, Z21 und Z23 in der Schilddrüsengewebe- und/oder -zellprobe in Zellkernen mit einem Verlust der Prägung und/oder einer Kopienanzahlvariation exprimiert sind, wird die Schilddrüsengewebe- und/oder -zellprobe als eine Schilddrüsenkrebsgewebe- und/oder -zellprobe bestimmt;
oder
(m) Unterziehen einer Blasengewebe- und/oder -zellprobe einer Analyse der Expression der geprägten Gene Z18, Z19, Z20, Z21, Z22, Z26, Z27 und Z28, so dass:
falls die geprägten Gene Z18, Z19, Z20, Z21, Z22, Z26, Z27 und Z28 in der Blasengewebe-und/oder -zellprobe in Zellkernen nicht exprimiert sind oder normal exprimiert sind, wird die Blasengewebe- und/oder -zellprobe als eine gesunde Blasengewebe- und/oder -zellprobe bestimmt; und
falls die geprägten Gene Z18, Z19, Z20, Z21, Z22, Z26, Z27 und Z28 in der Blasengewebe-und/oder -zellprobe in Zellkernen mit einem Verlust der Prägung und/oder einer Kopienanzahlvariation exprimiert sind, wird die Blasengewebe- und/oder -zellprobe als eine Blasenkrebsgewebe- und/oder -zellprobe bestimmt;
oder
(n) Unterziehen einer Lebergewebe- und/oder -zellprobe einer Analyse der Expression der geprägten Gene Z17, Z19, Z21, Z22, Z26, Z27 und Z28, so dass:
falls die geprägten Gene Z17, Z19, Z21, Z22, Z26, Z27 und Z28 in der Lebergewebe-und/oder -zellprobe in Zellkernen nicht exprimiert sind oder normal exprimiert sind, wird die Lebergewebe- und/oder -zellprobe als eine gesunde Lebergewebe- und/oder -zellprobe bestimmt; und
falls die geprägten Gene Z17, Z19, Z21, Z22, Z26, Z27 und Z28 in der Lebergewebe-und/oder -zellprobe in Zellkernen mit einem Verlust der Prägung und/oder einer Kopienanzahlvariation exprimiert sind, wird die Lebergewebe- und/oder -zellprobe als eine Leberkrebsgewebe- und/oder -zellprobe bestimmt;
oder
(o) Unterziehen einer Bauchspeicheldrüsengewebe- und/oder -zellprobe einer Analyse der Expression der geprägten Gene Z17, Z18, Z19, Z20, Z21, Z22, Z26, Z27 und Z28, so dass:
falls die geprägten Gene Z17, Z18, Z19, Z20, Z21, Z22, Z26, Z27 und Z28 in der Bauchspeicheldrüsengewebe- und/oder -zellprobe in Zellkernen nicht exprimiert sind oder normal exprimiert sind, wird die Bauchspeicheldrüsengewebe- und/oder -zellprobe als eine gesunde Bauchspeicheldrüsengewebe- und/oder -zellprobe bestimmt; und
falls die geprägten Gene Z17, Z18, Z19, Z20, Z21, Z22, Z26, Z27 und Z28 in der Lebergewebe- und/oder -zellprobe in Zellkernen mit einem Verlust der Prägung und/oder einer Kopienanzahlvariation exprimiert sind, wird die Bauchspeicheldrüsengewebe- und/oder -zellprobe als eine Bauchspeicheldrüsenkrebsgewebe- und/oder -zellprobe bestimmt;
oder
(p) Unterziehen einer lymphatischen Gewebe- und/oder -zellprobe einer Analyse der Expression der geprägten Gene Z18, Z19, Z21, Z22, Z26, Z27 und Z28, so dass:
falls die geprägten Gene Z18, Z19, Z21, Z22, Z26, Z27 und Z28 in der lymphatischen Gewebe- und/oder -zellprobe in Zellkernen nicht exprimiert sind oder normal exprimiert sind, wird die lymphatische Gewebe- und/oder -zellprobe als eine gesunde lymphatische Gewebe- und/oder -zellprobe bestimmt; und
falls die geprägten Gene Z18, Z19, Z21, Z22, Z26, Z27 und Z28 in der lymphatischen Gewebe- und/oder -zellprobe in Zellkernen mit einem Verlust der Prägung und/oder einer Kopienanzahlvariation exprimiert sind, wird die lymphatische Gewebe- und/oder -zellprobe als eine Lymphomgewebe- und/oder -zellprobe bestimmt;
oder
(q) Unterziehen einer kolorektalen Gewebe- und/oder -zellprobe einer Analyse der Expression der geprägten Gene Z19, Z20, Z21, Z22, Z26 und Z27, so dass:
falls die geprägten Gene Z19, Z20, Z21, Z22, Z26 und Z27in der kolorektalen Gewebe-und/oder -zellprobe in Zellkernen nicht exprimiert sind oder normal exprimiert sind, wird die kolorektale Gewebe- und/oder -zellprobe als eine gesunde kolorektale Gewebe- und/oder -zellprobe bestimmt; und
falls die geprägten Gene Z19, Z20, Z21, Z22, Z26 und Z27in der kolorektalen Gewebe-und/oder -zellprobe in Zellkernen mit einem Verlust der Prägung und/oder einer Kopienanzahlvariation exprimiert sind, wird die kolorektale Gewebe- und/oder -zellprobe als eine Darmkrebsgewebe- und/oder -zellprobe bestimmt;
oder
(r) Unterziehen einer Hautgewebe- und/oder -zellprobe einer Analyse der Expression der geprägten Gene Z18, Z19, Z21, Z22, Z25 und Z28, so dass:
falls die geprägten Gene Z18, Z19, Z21, Z22, Z25 und Z28 in der Hautgewebe- und/oder -zellprobe in Zellkernen nicht exprimiert sind oder normal exprimiert sind, wird die Hautgewebe-und/oder -zellprobe als eine gesunde Hautgewebe- und/oder -zellprobe bestimmt; und
falls die geprägten Gene Z18, Z19, Z21, Z22, Z25 und Z28 in der Hautgewebe- und/oder -zellprobe in Zellkernen mit einem Verlust der Prägung und/oder einer Kopienanzahlvariation exprimiert sind, wird die Hautgewebe- und/oder -zellprobe als eine Hautkrebsgewebe- und/oder -zellprobe bestimmt;
oder
(s) Unterziehen einer Lungengewebe- und/oder -zellprobe einer Analyse der Expression der geprägten Gene Z18, Z19, Z21, Z22, Z25, Z26, Z27 und Z28, so dass:
falls die geprägten Gene Z18, Z19, Z21, Z22, Z25, Z26, Z27 und Z28 in der Lungengewebe-und/oder -zellprobe in Zellkernen nicht exprimiert sind oder normal exprimiert sind, wird die Lungengewebe- und/oder -zellprobe als eine gesunde Lungengewebe- und/oder -zellprobe bestimmt; und
falls die geprägten Gene Z18, Z19, Z21, Z22, Z25, Z26, Z27 und Z28 in der Lungengewebe-und/oder -zellprobe in Zellkernen mit einem Verlust der Prägung und/oder einer Kopienanzahlvariation exprimiert sind, wird die Lungengewebe- und/oder -zellprobe als eine Lungenkrebsgewebe- und/oder -zellprobe bestimmt.

## Revendications

1. Procédé de diagnostic de tumeur comprenant :
(a) la soumission d'un échantillon de tissu et/ou de cellules des voies biliaires à une analyse de l'expression des gènes soumis à empreinte Z1, Z2, Z8, Z10, N11, Z14, N16, Z19, Z20, Z21, Z22, Z25, Z26, et Z27 de telle sorte que :
si les gènes soumis à empreinte Z1, Z2, Z8, Z10, N11, Z14, N16, Z19, Z20, Z21, Z22, Z25, Z26, et Z27 ne sont pas exprimés ou sont exprimés normalement dans les noyaux cellulaires dans l'échantillon de tissu et/ou de cellules des voies biliaires, l'échantillon de tissu et/ou de cellules des voies biliaires est déterminé comme étant un échantillon sain de tissu et/ou de cellules des voies biliaires ; et
si le gène soumis à empreinte Z1, Z2, Z8, Z10, N11, Z14, N16, Z19, Z20, Z21, Z22, Z25, Z26, ou Z27 est exprimé avec une perte d'empreinte et/ou une variation du nombre de copies dans les noyaux cellulaires dans l'échantillon de tissu et/ou de cellules des voies biliaires, l'échantillon de tissu et/ou de cellules des voies biliaires est déterminé comme étant un échantillon de tissu et/ou de cellules de cholangiocarcinome ;
ou
(b) la soumission d'un échantillon de tissu cérébral et/ou de cellules cérébrales à une analyse de l'expression des gènes soumis à empreinte Z1, Z2, Z4, Z5, Z8, Z9, Z10, N11, Z13, Z14, N16, Z17, Z19, Z21, Z22, Z23, Z26, Z27 et Z28 de telle sorte que :
si les gènes soumis à empreinte Z1, Z2, Z4, Z5, Z8, Z9, Z10, N11, Z13, Z14, N16, Z17, Z19, Z21, Z22, Z23, Z26, Z27 et Z28 ne sont pas exprimés ou sont exprimés normalement dans les noyaux cellulaires dans l'échantillon de tissu cérébral et/ou de cellules cérébrales, l'échantillon de tissu cérébral et/ou de cellules cérébrales est déterminé comme étant un échantillon sain de tissu cérébral et/ou de cellules cérébrales ; et
si le gène soumis à empreinte Z1, Z2, Z4, Z5, Z8, Z9, Z10, N11, Z13, Z14, N16, Z17, Z19, Z21, Z22, Z23, Z26, Z27 ou Z28 est exprimé avec une perte d'empreinte et/ou une variation du nombre de copies dans les noyaux cellulaires dans l'échantillon de tissu cérébral et/ou de cellules cérébrales, l'échantillon de tissu cérébral et/ou de cellules cérébrales est déterminé comme étant un échantillon de cancer de tissu cérébral et/ou de cellules cérébrales ;
ou
(c) la soumission d'un échantillon de tissu et/ou de cellules de rein à une analyse de l'expression des gènes soumis à empreinte Z1, Z4, Z6, Z8, Z10, N11, Z13, N16, Z17, Z18, Z19, Z20, Z21, Z25, Z26, Z27, et Z28 de telle sorte que :
si les gènes soumis à empreinte Z1, Z4, Z6, Z8, Z10, N11, Z13, N16, Z17, Z18, Z19, Z20, Z21, Z25, Z26, Z27, et Z28 ne sont pas exprimés ou sont exprimés normalement dans les noyaux cellulaires dans l'échantillon de tissu et/ou de cellules de rein, l'échantillon de tissu et/ou de cellules de rein est déterminé comme étant un échantillon sain de tissu et/ou de cellules de rein ; et
si le gène soumis à empreinte Z1, Z4, Z6, Z8, Z10, N11, Z13, N16, Z17, Z18, Z19, Z20, Z21, Z25, Z26, Z27, ou Z28 est exprimé avec une perte d'empreinte et/ou une variation du nombre de copies dans les noyaux cellulaires dans l'échantillon de tissu et/ou de cellules de rein, l'échantillon de tissu et/ou de cellules de rein est déterminé comme étant un échantillon de cancer de tissu et/ou de cellules de rein ;
ou
(d) la soumission d'un échantillon de tissu osseux et/ou de cellules osseuses à une analyse de l'expression des gènes soumis à empreinte Z1, Z2, Z3, Z6, Z8, Z9, Z10, N11, Z13, N16, Z18, Z19, Z20, Z21, Z22, Z25, Z26, Z27 et Z28 de telle sorte que :
si les gènes soumis à empreinte Z1, Z2, Z3, Z6, Z8, Z9, Z10, N11, Z13, N16, Z18, Z19, Z20, Z21, Z22, Z25, Z26, Z27 et Z28 ne sont pas exprimés ou sont exprimés normalement dans les noyaux cellulaires dans l'échantillon de tissu osseux et/ou de cellules osseuses, l'échantillon de tissu osseux et/ou de cellules osseuses est déterminé comme étant un échantillon sain de tissu osseux et/ou de cellules osseuses ; et
si le gène soumis à empreinte Z1, Z2, Z3, Z6, Z8, Z9, Z10, N11, Z13, N16, Z18, Z19, Z20, Z21, Z22, Z25, Z26, Z27 ou Z28 est exprimé avec une perte d'empreinte et/ou une variation du nombre de copies dans les noyaux cellulaires dans l'échantillon de tissu osseux et/ou de cellules osseuses, l'échantillon de tissu osseux et/ou de cellules osseuses est déterminé comme étant un échantillon de cancer de tissu osseux et/ou de cellules osseuses ;
ou
(e) la soumission d'un échantillon de tissu et/ou de cellules de col de l'utérus à une analyse de l'expression des gènes soumis à empreinte Z1, Z3, Z5, Z8, Z9, Z10, N11, N16, Z17, Z19, Z20, Z21, Z22, Z23, Z26, Z27, et Z28 de telle sorte que :
si les gènes soumis à empreinte Z1, Z3, Z5, Z8, Z9, Z10, N11, N16, Z17, Z19, Z20, Z21, Z22, Z23, Z26, Z27, et Z28 ne sont pas exprimés ou sont exprimés normalement dans les noyaux cellulaires dans l'échantillon de tissu et/ou de cellules de col de l'utérus, l'échantillon de tissu et/ou de cellules de col de l'utérus est déterminé comme étant un échantillon sain de tissu et/ou de cellules de col de l'utérus ; et
si le gène soumis à empreinte Z1, Z3, Z5, Z8, Z9, Z10, N11, N16, Z17, Z19, Z20, Z21, Z22, Z23, Z26, Z27, ou Z28 est exprimé avec une perte d'empreinte et/ou une variation du nombre de copies dans les noyaux cellulaires dans l'échantillon de tissu et/ou de cellules de col de l'utérus, l'échantillon de tissu et/ou de cellules de col de l'utérus est déterminé comme étant un échantillon de cancer de tissu et/ou de cellules de col de l'utérus ;
ou
(f) la soumission d'un échantillon de tissu et/ou de cellules de rhinopharynx à une analyse de l'expression des gènes soumis à empreinte Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 et Z28 de telle sorte que :
si les gènes soumis à empreinte Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 et Z28 ne sont pas exprimés ou sont exprimés normalement dans les noyaux cellulaires dans l'échantillon de tissu et/ou de cellules de rhinopharynx, l'échantillon de tissu et/ou de cellules de rhinopharynx est déterminé comme étant un échantillon sain de tissu et/ou de cellules de rhinopharynx ; et
si le gène soumis à empreinte Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 ou Z28 est exprimé avec une perte d'empreinte et/ou une variation du nombre de copies dans les noyaux cellulaires dans l'échantillon de tissu et/ou de cellules de rhinopharynx, l'échantillon de tissu et/ou de cellules de rhinopharynx est déterminé comme étant un échantillon de tissu et/ou de cellules de carcinome de rhinopharynx ;
ou
(g) la soumission d'un échantillon de tissu et/ou de cellules de larynx à une analyse de l'expression des gènes soumis à empreinte Z1, Z3, Z6, Z8, N11, Z15, N16, Z17, Z18, Z19, Z21, Z22, Z25, Z26, Z27 et Z28 de telle sorte que :
si les gènes soumis à empreinte Z1, Z3, Z6, Z8, N11, Z15, N16, Z17, Z18, Z19, Z21, Z22, Z25, Z26, Z27 et Z28 ne sont pas exprimés ou sont exprimés normalement dans les noyaux cellulaires dans l'échantillon de tissu et/ou de cellules de larynx, l'échantillon de tissu et/ou de cellules de larynx est déterminé comme étant un échantillon sain de tissu et/ou de cellules de larynx ; et
si le gène soumis à empreinte Z1, Z3, Z6, Z8, N11, Z15, N16, Z17, Z18, Z19, Z21, Z22, Z25, Z26, Z27 ou Z28 est exprimé avec une perte d'empreinte et/ou une variation du nombre de copies dans les noyaux cellulaires dans l'échantillon de tissu et/ou de cellules de larynx, l'échantillon de tissu et/ou de cellules de larynx est déterminé comme étant un échantillon de tissu et/ou de cellules de cancer du larynx ;
ou
(h) la soumission d'un échantillon de tissu et/ou de cellules de prostate à une analyse de l'expression des gènes soumis à empreinte Z18, Z19, Z20, Z21, Z25, Z26, Z27 et Z28 de telle sorte que :
si les gènes soumis à empreinte Z18, Z19, Z20, Z21, Z25, Z26, Z27 et Z28 ne sont pas exprimés ou sont exprimés normalement dans les noyaux cellulaires dans l'échantillon de tissu et/ou de cellules de prostate, l'échantillon de tissu et/ou de cellules de prostate est déterminé comme étant un échantillon sain de tissu et/ou de cellules de prostate ; et
si le gène soumis à empreinte Z18, Z19, Z20, Z21, Z25, Z26, Z27 ou Z28 est exprimé avec une perte d'empreinte et/ou une variation du nombre de copies dans les noyaux cellulaires dans l'échantillon de tissu et/ou de cellules de prostate, l'échantillon de tissu et/ou de cellules de prostate est déterminé comme étant un échantillon de tissu et/ou de cellules de cancer de la prostate ;
ou
(i) la soumission d'un échantillon de tissu gastrique et/ou de cellules gastriques à une analyse de l'expression des gènes soumis à empreinte Z18, Z19, Z20, Z21, Z22, Z26 et Z27 de telle sorte que :
si les gènes soumis à empreinte Z18, Z19, Z20, Z21, Z22, Z26 et Z27 ne sont pas exprimés ou sont exprimés normalement dans les noyaux cellulaires dans l'échantillon de tissu gastrique et/ou de cellules gastriques, l'échantillon de tissu gastrique et/ou de cellules gastriques est déterminé comme étant un échantillon sain de tissu gastrique et/ou de cellules gastriques ; et
si le gène soumis à empreinte Z18, Z19, Z20, Z21, Z22, Z26 ou Z27 est exprimé avec une perte d'empreinte et/ou une variation du nombre de copies dans les noyaux cellulaires dans l'échantillon de tissu gastrique et/ou de cellules gastriques, l'échantillon de tissu gastrique et/ou de cellules gastriques est déterminé comme étant un échantillon de tissu et/ou de cellules de carcinome gastrique ;
ou
(j) la soumission d'un échantillon de tissu et/ou de cellules d'œsophage à une analyse de l'expression des gènes soumis à empreinte Z18, Z19, Z21, Z22, Z26, Z27 et Z28 de telle sorte que :
si les gènes soumis à empreinte Z18, Z19, Z21, Z22, Z26, Z27 et Z28 ne sont pas exprimés ou sont exprimés normalement dans les noyaux cellulaires dans l'échantillon de tissu et/ou de cellules d'œsophage, l'échantillon de tissu et/ou de cellules d'œsophage est déterminé comme étant un échantillon sain de tissu et/ou de cellules d'œsophage ; et
si le gène soumis à empreinte Z18, Z19, Z21, Z22, Z26, Z27 ou Z28 est exprimé avec une perte d'empreinte et/ou une variation du nombre de copies dans les noyaux cellulaires dans l'échantillon de tissu et/ou de cellules d'œsophage, l'échantillon de tissu et/ou de cellules d'œsophage est déterminé comme étant un échantillon de tissu et/ou de cellules de cancer d'œsophage ;
ou
(k) la soumission d'un échantillon de tissu mammaire et/ou de cellules mammaires à une analyse de l'expression des gènes soumis à empreinte Z18, Z19, Z21, Z22 et Z27 de telle sorte que :
si les gènes soumis à empreinte Z18, Z19, Z21, Z22 et Z27 ne sont pas exprimés ou sont exprimés normalement dans les noyaux cellulaires dans l'échantillon de tissu mammaire et/ou de cellules mammaires, l'échantillon de tissu mammaire et/ou de cellules mammaires est déterminé comme étant un échantillon sain de tissu mammaire et/ou de cellules mammaires ; et
si le gène soumis à empreinte Z18, Z19, Z21, Z22 ou Z27 est exprimé avec une perte d'empreinte et/ou une variation du nombre de copies dans les noyaux cellulaires dans l'échantillon de tissu mammaire et/ou de cellules mammaires, l'échantillon de tissu mammaire et/ou de cellules mammaires est déterminé comme étant un échantillon de tissu et/ou de cellules de cancer mammaire ;
ou
(l) la soumission d'un échantillon de tissu et/ou de cellules de thyroïde à une analyse de l'expression des gènes soumis à empreinte Z18, Z19, Z20, Z21 et Z23 de telle sorte que :
si les gènes soumis à empreinte Z18, Z19, Z20, Z21 et Z23 ne sont pas exprimés ou sont exprimés normalement dans les noyaux cellulaires dans l'échantillon de tissu et/ou de cellules de thyroïde, l'échantillon de tissu et/ou de cellules de thyroïde est déterminé comme étant un échantillon sain de tissu et/ou de cellules de thyroïde ; et
si le gène soumis à empreinte Z18, Z19, Z20, Z21 ou Z23 est exprimé avec une perte d'empreinte et/ou une variation du nombre de copies dans les noyaux cellulaires dans l'échantillon de tissu et/ou de cellules de thyroïde, l'échantillon de tissu et/ou de cellules de thyroïde est déterminé comme étant un échantillon de tissu et/ou de cellules de cancer de la thyroïde ;
ou
(m) la soumission d'un échantillon de tissu et/ou de cellules de vessie à une analyse de l'expression des gènes soumis à empreinte Z18, Z19, Z20, Z21, Z22, Z26, Z27 et Z28 de telle sorte que :
si les gènes soumis à empreinte Z18, Z19, Z20, Z21, Z22, Z26, Z27 et Z28 ne sont pas exprimés ou sont exprimés normalement dans les noyaux cellulaires dans l'échantillon de tissu et/ou de cellules de vessie, l'échantillon de tissu et/ou de cellules de vessie est déterminé comme étant un échantillon sain de tissu et/ou de cellules de vessie ; et
si le gène soumis à empreinte Z18, Z19, Z20, Z21, Z22, Z26, Z27 ou Z28 est exprimé avec une perte d'empreinte et/ou une variation du nombre de copies dans les noyaux cellulaires dans l'échantillon de tissu et/ou de cellules de vessie, l'échantillon de tissu et/ou de cellules de vessie est déterminé comme étant un échantillon de tissu et/ou de cellules de cancer de la vessie ;
ou
(n) la soumission d'un échantillon de tissu et/ou de cellules de foie à une analyse de l'expression des gènes soumis à empreinte Z17, Z19, Z21, Z22, Z26, Z27 et Z28 de telle sorte que :
si les gènes soumis à empreinte Z17, Z19, Z21, Z22, Z26, Z27 et Z28 ne sont pas exprimés ou sont exprimés normalement dans les noyaux cellulaires dans l'échantillon de tissu et/ou de cellules de foie, l'échantillon de tissu et/ou de cellules de foie est déterminé comme étant un échantillon sain de tissu et/ou de cellules de foie ; et
si le gène soumis à empreinte Z17, Z19, Z21, Z22, Z26, Z27 ou Z28 est exprimé avec une perte d'empreinte et/ou une variation du nombre de copies dans les noyaux cellulaires dans l'échantillon de tissu et/ou de cellules de foie, l'échantillon de tissu et/ou de cellules de foie est déterminé comme étant un échantillon de tissu et/ou de cellules de cancer du foie ;
ou
(o) la soumission d'un échantillon de tissu et/ou de cellules de pancréas à une analyse de l'expression des gènes soumis à empreinte Z17, Z18, Z19, Z20, Z21, Z22, Z26, Z27, et Z28 de telle sorte que :
si les gènes soumis à empreinte Z17, Z18, Z19, Z20, Z21, Z22, Z26, Z27, et Z28 ne sont pas exprimés ou sont exprimés normalement dans les noyaux cellulaires dans l'échantillon de tissu et/ou de cellules de pancréas, l'échantillon de tissu et/ou de cellules de pancréas est déterminé comme étant un échantillon sain de tissu et/ou de cellules de pancréas ; et
si le gène soumis à empreinte Z17, Z18, Z19, Z20, Z21, Z22, Z26, Z27, ou Z28 est exprimé avec une perte d'empreinte et/ou une variation du nombre de copies dans les noyaux cellulaires dans l'échantillon de tissu et/ou de cellules de pancréas, l'échantillon de tissu et/ou de cellules de pancréas est déterminé comme étant un échantillon de tissu et/ou de cellules de cancer du pancréas ;
ou
(p) la soumission d'un échantillon de tissu lymphatique et/ou de cellules lymphatiques à une analyse de l'expression des gènes soumis à empreinte Z18, Z19, Z21, Z22, Z26, Z27 et Z28 de telle sorte que :
si les gènes soumis à empreinte Z18, Z19, Z21, Z22, Z26, Z27 et Z28 ne sont pas exprimés ou sont exprimés normalement dans les noyaux cellulaires dans l'échantillon de tissu lymphatique et/ou de cellules lymphatiques, l'échantillon de tissu lymphatique et/ou de cellules lymphatiques est déterminé comme étant un échantillon sain de tissu lymphatique et/ou de cellules lymphatiques ; et
si le gène soumis à empreinte Z18, Z19, Z21, Z22, Z26, Z27 ou Z28 est exprimé avec une perte d'empreinte et/ou une variation du nombre de copies dans les noyaux cellulaires dans l'échantillon de tissu lymphatique et/ou de cellules lymphatiques, l'échantillon de tissu lymphatique et/ou de cellules lymphatiques est déterminé comme étant un échantillon de tissu et/ou de cellules de lymphome ;
ou
(q) la soumission d'un échantillon de tissu colorectal et/ou de cellules colorectales à une analyse de l'expression des gènes soumis à empreinte Z19, Z20, Z21, Z22, Z26 et Z27 de telle sorte que :
si les gènes soumis à empreinte Z19, Z20, Z21, Z22, Z26 et Z27 ne sont pas exprimés ou sont exprimés normalement dans les noyaux cellulaires dans l'échantillon de tissu colorectal et/ou de cellules colorectales, l'échantillon de tissu colorectal et/ou de cellules colorectales est déterminé comme étant un échantillon sain de tissu colorectal et/ou de cellules colorectales ; et
si le gène soumis à empreinte Z19, Z20, Z21, Z22, Z26 ou Z27 est exprimé avec une perte d'empreinte et/ou une variation du nombre de copies dans les noyaux cellulaires dans l'échantillon de tissu colorectal et/ou de cellules colorectales, l'échantillon de tissu colorectal et/ou de cellules colorectales est déterminé comme étant un échantillon de tissu et/ou de cellules de cancer colorectal ;
ou
(r) la soumission d'un échantillon de tissu et/ou de cellules de peau à une analyse de l'expression des gènes soumis à empreinte Z18, Z19, Z21, Z22, Z25 et Z28 de telle sorte que :
si les gènes soumis à empreinte Z18, Z19, Z21, Z22, Z25 et Z28 ne sont pas exprimés ou sont exprimés normalement dans les noyaux cellulaires dans l'échantillon de tissu et/ou de cellules de peau, l'échantillon de tissu et/ou de cellules de peau est déterminé comme étant un échantillon sain de tissu et/ou de cellules de peau ; et
si le gène soumis à empreinte Z18, Z19, Z21, Z22, Z25 ou Z28 est exprimé avec une perte d'empreinte et/ou une variation du nombre de copies dans les noyaux cellulaires dans l'échantillon de tissu et/ou de cellules de peau, l'échantillon de tissu et/ou de cellules de peau est déterminé comme étant un échantillon de tissu et/ou de cellules de cancer de la peau ;
ou
(s) la soumission d'un échantillon de tissu et/ou de cellules de poumon à une analyse de l'expression des gènes soumis à empreinte Z18, Z19, Z21, Z22, Z25, Z26, Z27 et Z28 de telle sorte que :
si les gènes soumis à empreinte Z18, Z19, Z21, Z22, Z25, Z26, Z27 et Z28 ne sont pas exprimés ou sont exprimés normalement dans les noyaux cellulaires dans l'échantillon de tissu et/ou de cellules de poumon, l'échantillon de tissu et/ou de cellules de poumon est déterminé comme étant un échantillon sain de tissu et/ou de cellules de poumon ; et
si le gène soumis à empreinte Z18, Z19, Z21, Z22, Z25, Z26, Z27 ou Z28 est exprimé avec une perte d'empreinte et/ou une variation du nombre de copies dans les noyaux cellulaires dans l'échantillon de tissu et/ou de cellules de poumon, l'échantillon de tissu et/ou de cellules de poumon est déterminé comme étant un échantillon de tissu et/ou de cellules de cancer du poumon ;
dans lequel le gène soumis à empreinte Z1 est Gnas, le gène soumis à empreinte Z2 est Igf2, le gène soumis à empreinte Z3 est Peg10, le gène soumis à empreinte Z4 est Igf2r, le gène soumis à empreinte Z5 est Mest, le gène soumis à empreinte Z6 est Plagl1, le gène soumis à empreinte Z8 est Dcn, le gène soumis à empreinte Z9 est DIk1, le gène soumis à empreinte Z10 est Gatm, le gène soumis à empreinte N11 est Grb10, le gène soumis à empreinte Z12 est Peg3, le gène soumis à empreinte Z13 est Sgce, le gène soumis à empreinte Z14 est Slc38a4, le gène soumis à empreinte Z15 est Diras3, le gène soumis à empreinte N16 est Snrpn/Snurf, le gène soumis à empreinte Z17 est Aldh1l1, le gène soumis à empreinte Z18 est Gata3, le gène soumis à empreinte Z19 est Hm13, le gène soumis à empreinte Z20 est Kcnq1, le gène soumis à empreinte Z21 est Nhp2l1, le gène soumis à empreinte Z22 est Pon2, le gène soumis à empreinte Z23 est Rasgrf1, le gène soumis à empreinte Z25 est Tfpi2, le gène soumis à empreinte Z26 est Trappc9, le gène soumis à empreinte Z27 est Ube3a, et le gène soumis à empreinte Z28 est Zic1.

2. Procédé selon la revendication 1, dans lequel l'échantillon est issu de tissus et/ou de cellules.

3. Procédé selon la revendication 2, dans lequel l'hybridation in situ utilise un procédé d'hybridation in situ RNAscope.

4. Procédé selon la revendication 3, dans lequel le procédé d'hybridation in situ RNAscope utilise un kit de réactif chromogène monocanal ou multicanal ou un kit de réactif fluorescent monocanal ou multicanal.

5. Procédé selon la revendication 4, dans lequel le procédé d'hybridation in situ RNAscope utilise un kit de réactif chromogène rouge/marron monocanal ou un kit de réactif fluorescent multicanal.

6. Procédé selon la revendication 4 ou la revendication 5, dans lequel :
ledit gène soumis à empreinte est déterminé comme n'étant pas exprimé lorsqu'aucun marqueur de gène soumis à empreinte n'est présent dans le noyau d'une cellule de l'échantillon ;
le gène soumis à empreinte est déterminé comme étant exprimé normalement lorsqu'un seul marqueur de gène soumis à empreinte est présent dans le noyau de la cellule de l'échantillon ;
le gène soumis à empreinte est déterminé comme ayant une perte d'empreinte lorsque deux marqueurs de gène soumis à empreinte sont présents dans le noyau de la cellule de l'échantillon ; et
le gène soumis à empreinte est déterminé comme ayant une variation de numéro de copies lorsque plus de deux marqueurs de gène soumis à empreinte sont présents dans le noyau de la cellule de l'échantillon.

7. Dispositif de diagnostic de tumeur, comprenant :
une unité d'échantillonnage pour obtenir un échantillon ;
une unité de conception de sonde pour concevoir une sonde spécifique à la séquence d'un gène soumis à empreinte à détecter;
une unité de détection pour réaliser une hybridation in situ entre la sonde et l'échantillon ; et
une unité d'analyse pour analyser l'expression du gène soumis à empreinte par imagerie microscopique ;
dans lequel le dispositif de diagnostic de tumeur est configuré pour effectuer un diagnostic de tumeur en utilisant un procédé d'analyse comprenant :
(a) la soumission d'un échantillon de tissu et/ou de cellules des voies biliaires à une analyse de l'expression des gènes soumis à empreinte Z1, Z2, Z8, Z10, N11, Z14, N16, Z19, Z20, Z21, Z22, Z25, Z26, et Z27 de telle sorte que :
si les gènes soumis à empreinte Z1, Z2, Z8, Z10, N11, Z14, N16, Z19, Z20, Z21, Z22, Z25, Z26, et Z27 ne sont pas exprimés ou sont exprimés normalement dans les noyaux cellulaires dans l'échantillon de tissu et/ou de cellules des voies biliaires, l'échantillon de tissu et/ou de cellules des voies biliaires est déterminé comme étant un échantillon sain de tissu et/ou de cellules des voies biliaires ; et
si le gène soumis à empreinte Z1, Z2, Z8, Z10, N11, Z14, N16, Z19, Z20, Z21, Z22, Z25, Z26, ou Z27 est exprimé avec une perte d'empreinte et/ou une variation du nombre de copies dans les noyaux cellulaires dans l'échantillon de tissu et/ou de cellules des voies biliaires, l'échantillon de tissu et/ou de cellules des voies biliaires est déterminé comme étant un échantillon de tissu et/ou de cellules de cholangiocarcinome ;
ou
(b) la soumission d'un échantillon de tissu cérébral et/ou de cellules cérébrales à une analyse de l'expression des gènes soumis à empreinte Z1, Z2, Z4, Z5, Z8, Z9, Z10, N11, Z13, Z14, N16, Z17, Z19, Z21, Z22, Z23, Z26, Z27 et Z28 de telle sorte que :
si les gènes soumis à empreinte Z1, Z2, Z4, Z5, Z8, Z9, Z10, N11, Z13, Z14, N16, Z17, Z19, Z21, Z22, Z23, Z26, Z27 et Z28 ne sont pas exprimés ou sont exprimés normalement dans les noyaux cellulaires dans l'échantillon de tissu cérébral et/ou de cellules cérébrales, l'échantillon de tissu cérébral et/ou de cellules cérébrales est déterminé comme étant un échantillon sain de tissu cérébral et/ou de cellules cérébrales ; et
si le gène soumis à empreinte Z1, Z2, Z4, Z5, Z8, Z9, Z10, N11, Z13, Z14, N16, Z17, Z19, Z21, Z22, Z23, Z26, Z27 ou Z28 est exprimé avec une perte d'empreinte et/ou une variation du nombre de copies dans les noyaux cellulaires dans l'échantillon de tissu cérébral et/ou de cellules cérébrales, l'échantillon de tissu cérébral et/ou de cellules cérébrales est déterminé comme étant un échantillon de cancer de tissu cérébral et/ou de cellules cérébrales ;
ou
(c) la soumission d'un échantillon de tissu et/ou de cellules de rein à une analyse de l'expression des gènes soumis à empreinte Z1, Z4, Z6, Z8, Z10, N11, Z13, N16, Z17, Z18, Z19, Z20, Z21, Z25, Z26, Z27, et Z28 de telle sorte que :
si les gènes soumis à empreinte Z1, Z4, Z6, Z8, Z10, N11, Z13, N16, Z17, Z18, Z19, Z20, Z21, Z25, Z26, Z27, et Z28 ne sont pas exprimés ou sont exprimés normalement dans les noyaux cellulaires dans l'échantillon de tissu et/ou de cellules de rein, l'échantillon de tissu et/ou de cellules de rein est déterminé comme étant un échantillon sain de tissu et/ou de cellules de rein ; et
si le gène soumis à empreinte Z1, Z4, Z6, Z8, Z10, N11, Z13, N16, Z17, Z18, Z19, Z20, Z21, Z25, Z26, Z27, ou Z28 est exprimé avec une perte d'empreinte et/ou une variation du nombre de copies dans les noyaux cellulaires dans l'échantillon de tissu et/ou de cellules de rein, l'échantillon de tissu et/ou de cellules de rein est déterminé comme étant un échantillon de cancer de tissu et/ou de cellules de rein ;
ou
(d) la soumission d'un échantillon de tissu osseux et/ou de cellules osseuses à une analyse de l'expression des gènes soumis à empreinte Z1, Z2, Z3, Z6, Z8, Z9, Z10, N11, Z13, N16, Z18, Z19, Z20, Z21, Z22, Z25, Z26, Z27 et Z28 de telle sorte que :
si les gènes soumis à empreinte Z1, Z2, Z3, Z6, Z8, Z9, Z10, N11, Z13, N16, Z18, Z19, Z20, Z21, Z22, Z25, Z26, Z27 et Z28 ne sont pas exprimés ou sont exprimés normalement dans les noyaux cellulaires dans l'échantillon de tissu osseux et/ou de cellules osseuses, l'échantillon de tissu osseux et/ou de cellules osseuses est déterminé comme étant un échantillon sain de tissu osseux et/ou de cellules osseuses ; et
si le gène soumis à empreinte Z1, Z2, Z3, Z6, Z8, Z9, Z10, N11, Z13, N16, Z18, Z19, Z20, Z21, Z22, Z25, Z26, Z27 ou Z28 est exprimé avec une perte d'empreinte et/ou une variation du nombre de copies dans les noyaux cellulaires dans l'échantillon de tissu osseux et/ou de cellules osseuses, l'échantillon de tissu osseux et/ou de cellules osseuses est déterminé comme étant un échantillon de cancer de tissu osseux et/ou de cellules osseuses ;
ou
(e) la soumission d'un échantillon de tissu et/ou de cellules de col de l'utérus à une analyse de l'expression des gènes soumis à empreinte Z1, Z3, Z5, Z8, Z9, Z10, N11, N16, Z17, Z19, Z20, Z21, Z22, Z23, Z26, Z27, et Z28 de telle sorte que :
si les gènes soumis à empreinte Z1, Z3, Z5, Z8, Z9, Z10, N11, N16, Z17, Z19, Z20, Z21, Z22, Z23, Z26, Z27, et Z28 ne sont pas exprimés ou sont exprimés normalement dans les noyaux cellulaires dans l'échantillon de tissu et/ou de cellules de col de l'utérus, l'échantillon de tissu et/ou de cellules de col de l'utérus est déterminé comme étant un échantillon sain de tissu et/ou de cellules de col de l'utérus ; et
si le gène soumis à empreinte Z1, Z3, Z5, Z8, Z9, Z10, N11, N16, Z17, Z19, Z20, Z21, Z22, Z23, Z26, Z27, ou Z28 est exprimé avec une perte d'empreinte et/ou une variation du nombre de copies dans les noyaux cellulaires dans l'échantillon de tissu et/ou de cellules de col de l'utérus, l'échantillon de tissu et/ou de cellules de col de l'utérus est déterminé comme étant un échantillon de cancer de tissu et/ou de cellules de col de l'utérus ;
ou
(f) la soumission d'un échantillon de tissu et/ou de cellules de rhinopharynx à une analyse de l'expression des gènes soumis à empreinte Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 et Z28 de telle sorte que :
si les gènes soumis à empreinte Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 et Z28 ne sont pas exprimés ou sont exprimés normalement dans les noyaux cellulaires dans l'échantillon de tissu et/ou de cellules de rhinopharynx, l'échantillon de tissu et/ou de cellules de rhinopharynx est déterminé comme étant un échantillon sain de tissu et/ou de cellules de rhinopharynx ; et
si le gène soumis à empreinte Z1, Z3, Z4, Z5, Z6, Z8, Z9, Z10, N11, Z12, Z13, Z14, N16, Z17, Z18, Z19, Z20, Z21, Z22, Z23, Z25, Z26, Z27 ou Z28 est exprimé avec une perte d'empreinte et/ou une variation du nombre de copies dans les noyaux cellulaires dans l'échantillon de tissu et/ou de cellules de rhinopharynx, l'échantillon de tissu et/ou de cellules de rhinopharynx est déterminé comme étant un échantillon de tissu et/ou de cellules de carcinome de rhinopharynx ;
ou
(g) la soumission d'un échantillon de tissu et/ou de cellules de larynx à une analyse de l'expression des gènes soumis à empreinte Z1, Z3, Z6, Z8, N11, Z15, N16, Z17, Z18, Z19, Z21, Z22, Z25, Z26, Z27 et Z28 de telle sorte que :
si les gènes soumis à empreinte Z1, Z3, Z6, Z8, N11, Z15, N16, Z17, Z18, Z19, Z21, Z22, Z25, Z26, Z27 et Z28 ne sont pas exprimés ou sont exprimés normalement dans les noyaux cellulaires dans l'échantillon de tissu et/ou de cellules de larynx, l'échantillon de tissu et/ou de cellules de larynx est déterminé comme étant un échantillon sain de tissu et/ou de cellules de larynx ; et
si le gène soumis à empreinte Z1, Z3, Z6, Z8, N11, Z15, N16, Z17, Z18, Z19, Z21, Z22, Z25, Z26, Z27 ou Z28 est exprimé avec une perte d'empreinte et/ou une variation du nombre de copies dans les noyaux cellulaires dans l'échantillon de tissu et/ou de cellules de larynx, l'échantillon de tissu et/ou de cellules de larynx est déterminé comme étant un échantillon de tissu et/ou de cellules de cancer du larynx ;
ou
(h) la soumission d'un échantillon de tissu et/ou de cellules de prostate à une analyse de l'expression des gènes soumis à empreinte Z18, Z19, Z20, Z21, Z25, Z26, Z27 et Z28 de telle sorte que :
si les gènes soumis à empreinte Z18, Z19, Z20, Z21, Z25, Z26, Z27 et Z28 ne sont pas exprimés ou sont exprimés normalement dans les noyaux cellulaires dans l'échantillon de tissu et/ou de cellules de prostate, l'échantillon de tissu et/ou de cellules de prostate est déterminé comme étant un échantillon sain de tissu et/ou de cellules de prostate ; et
si le gène soumis à empreinte Z18, Z19, Z20, Z21, Z25, Z26, Z27 ou Z28 est exprimé avec une perte d'empreinte et/ou une variation du nombre de copies dans les noyaux cellulaires dans l'échantillon de tissu et/ou de cellules de prostate, l'échantillon de tissu et/ou de cellules de prostate est déterminé comme étant un échantillon de tissu et/ou de cellules de cancer de la prostate ;
ou
(i) la soumission d'un échantillon de tissu gastrique et/ou de cellules gastriques à une analyse de l'expression des gènes soumis à empreinte Z18, Z19, Z20, Z21, Z22, Z26 et Z27 de telle sorte que :
si les gènes soumis à empreinte Z18, Z19, Z20, Z21, Z22, Z26 et Z27 ne sont pas exprimés ou sont exprimés normalement dans les noyaux cellulaires dans l'échantillon de tissu gastrique et/ou de cellules gastriques, l'échantillon de tissu gastrique et/ou de cellules gastriques est déterminé comme étant un échantillon sain de tissu gastrique et/ou de cellules gastriques ; et
si le gène soumis à empreinte Z18, Z19, Z20, Z21, Z22, Z26 ou Z27 est exprimé avec une perte d'empreinte et/ou une variation du nombre de copies dans les noyaux cellulaires dans l'échantillon de tissu gastrique et/ou de cellules gastriques, l'échantillon de tissu gastrique et/ou de cellules gastriques est déterminé comme étant un échantillon de tissu et/ou de cellules de carcinome gastrique ;
ou
(j) la soumission d'un échantillon de tissu et/ou de cellules d'œsophage à une analyse de l'expression des gènes soumis à empreinte Z18, Z19, Z21, Z22, Z26, Z27 et Z28 de telle sorte que :
si les gènes soumis à empreinte Z18, Z19, Z21, Z22, Z26, Z27 et Z28 ne sont pas exprimés ou sont exprimés normalement dans les noyaux cellulaires dans l'échantillon de tissu et/ou de cellules d'œsophage, l'échantillon de tissu et/ou de cellules d'œsophage est déterminé comme étant un échantillon sain de tissu et/ou de cellules d'œsophage ; et
si le gène soumis à empreinte Z18, Z19, Z21, Z22, Z26, Z27 ou Z28 est exprimé avec une perte d'empreinte et/ou une variation du nombre de copies dans les noyaux cellulaires dans l'échantillon de tissu et/ou de cellules d'œsophage, l'échantillon de tissu et/ou de cellules d'œsophage est déterminé comme étant un échantillon de tissu et/ou de cellules de cancer d'œsophage ;
ou
(k) la soumission d'un échantillon de tissu mammaire et/ou de cellules mammaires à une analyse de l'expression des gènes soumis à empreinte Z18, Z19, Z21, Z22 et Z27 de telle sorte que :
si les gènes soumis à empreinte Z18, Z19, Z21, Z22 et Z27 ne sont pas exprimés ou sont exprimés normalement dans les noyaux cellulaires dans l'échantillon de tissu mammaire et/ou de cellules mammaires, l'échantillon de tissu mammaire et/ou de cellules mammaires est déterminé comme étant un échantillon sain de tissu mammaire et/ou de cellules mammaires ; et
si le gène soumis à empreinte Z18, Z19, Z21, Z22 ou Z27 est exprimé avec une perte d'empreinte et/ou une variation du nombre de copies dans les noyaux cellulaires dans l'échantillon de tissu mammaire et/ou de cellules mammaires, l'échantillon de tissu mammaire et/ou de cellules mammaires est déterminé comme étant un échantillon de tissu et/ou de cellules de cancer mammaire ;
ou
(l) la soumission d'un échantillon de tissu et/ou de cellules de thyroïde à une analyse de l'expression des gènes soumis à empreinte Z18, Z19, Z20, Z21 et Z23 de telle sorte que :
si les gènes soumis à empreinte Z18, Z19, Z20, Z21 et Z23 ne sont pas exprimés ou sont exprimés normalement dans les noyaux cellulaires dans l'échantillon de tissu et/ou de cellules de thyroïde, l'échantillon de tissu et/ou de cellules de thyroïde est déterminé comme étant un échantillon sain de tissu et/ou de cellules de thyroïde ; et
si le gène soumis à empreinte Z18, Z19, Z20, Z21 ou Z23 est exprimé avec une perte d'empreinte et/ou une variation du nombre de copies dans les noyaux cellulaires dans l'échantillon de tissu et/ou de cellules de thyroïde, l'échantillon de tissu et/ou de cellules de thyroïde est déterminé comme étant un échantillon de tissu et/ou de cellules de cancer de la thyroïde ;
ou
(m) la soumission d'un échantillon de tissu et/ou de cellules de vessie à une analyse de l'expression des gènes soumis à empreinte Z18, Z19, Z20, Z21, Z22, Z26, Z27 et Z28 de telle sorte que :
si les gènes soumis à empreinte Z18, Z19, Z20, Z21, Z22, Z26, Z27 et Z28 ne sont pas exprimés ou sont exprimés normalement dans les noyaux cellulaires dans l'échantillon de tissu et/ou de cellules de vessie, l'échantillon de tissu et/ou de cellules de vessie est déterminé comme étant un échantillon sain de tissu et/ou de cellules de vessie ; et
si le gène soumis à empreinte Z18, Z19, Z20, Z21, Z22, Z26, Z27 ou Z28 est exprimé avec une perte d'empreinte et/ou une variation du nombre de copies dans les noyaux cellulaires dans l'échantillon de tissu et/ou de cellules de vessie, l'échantillon de tissu et/ou de cellules de vessie est déterminé comme étant un échantillon de tissu et/ou de cellules de cancer de la vessie ;
ou
(n) la soumission d'un échantillon de tissu et/ou de cellules de foie à une analyse de l'expression des gènes soumis à empreinte Z17, Z19, Z21, Z22, Z26, Z27 et Z28 de telle sorte que :
si les gènes soumis à empreinte Z17, Z19, Z21, Z22, Z26, Z27 et Z28 ne sont pas exprimés ou sont exprimés normalement dans les noyaux cellulaires dans l'échantillon de tissu et/ou de cellules de foie, l'échantillon de tissu et/ou de cellules de foie est déterminé comme étant un échantillon sain de tissu et/ou de cellules de foie ; et
si le gène soumis à empreinte Z17, Z19, Z21, Z22, Z26, Z27 ou Z28 est exprimé avec une perte d'empreinte et/ou une variation du nombre de copies dans les noyaux cellulaires dans l'échantillon de tissu et/ou de cellules de foie, l'échantillon de tissu et/ou de cellules de foie est déterminé comme étant un échantillon de tissu et/ou de cellules de cancer du foie ;
ou
(o) la soumission d'un échantillon de tissu et/ou de cellules de pancréas à une analyse de l'expression des gènes soumis à empreinte Z17, Z18, Z19, Z20, Z21, Z22, Z26, Z27, et Z28 de telle sorte que :
si les gènes soumis à empreinte Z17, Z18, Z19, Z20, Z21, Z22, Z26, Z27, et Z28 ne sont pas exprimés ou sont exprimés normalement dans les noyaux cellulaires dans l'échantillon de tissu et/ou de cellules de pancréas, l'échantillon de tissu et/ou de cellules de pancréas est déterminé comme étant un échantillon sain de tissu et/ou de cellules de pancréas ; et
si le gène soumis à empreinte Z17, Z18, Z19, Z20, Z21, Z22, Z26, Z27, ou Z28 est exprimé avec une perte d'empreinte et/ou une variation du nombre de copies dans les noyaux cellulaires dans l'échantillon de tissu et/ou de cellules de pancréas, l'échantillon de tissu et/ou de cellules de pancréas est déterminé comme étant un échantillon de tissu et/ou de cellules de cancer du pancréas ;
ou
(p) la soumission d'un échantillon de tissu lymphatique et/ou de cellules lymphatiques à une analyse de l'expression des gènes soumis à empreinte Z18, Z19, Z21, Z22, Z26, Z27 et Z28 de telle sorte que :
si les gènes soumis à empreinte Z18, Z19, Z21, Z22, Z26, Z27 et Z28 ne sont pas exprimés ou sont exprimés normalement dans les noyaux cellulaires dans l'échantillon de tissu lymphatique et/ou de cellules lymphatiques, l'échantillon de tissu lymphatique et/ou de cellules lymphatiques est déterminé comme étant un échantillon sain de tissu lymphatique et/ou de cellules lymphatiques ; et
si le gène soumis à empreinte Z18, Z19, Z21, Z22, Z26, Z27 ou Z28 est exprimé avec une perte d'empreinte et/ou une variation du nombre de copies dans les noyaux cellulaires dans l'échantillon de tissu lymphatique et/ou de cellules lymphatiques, l'échantillon de tissu lymphatique et/ou de cellules lymphatiques est déterminé comme étant un échantillon de tissu et/ou de cellules de lymphome ;
ou
(q) la soumission d'un échantillon de tissu colorectal et/ou de cellules colorectales à une analyse de l'expression des gènes soumis à empreinte Z19, Z20, Z21, Z22, Z26 et Z27 de telle sorte que :
si les gènes soumis à empreinte Z19, Z20, Z21, Z22, Z26 et Z27 ne sont pas exprimés ou sont exprimés normalement dans les noyaux cellulaires dans l'échantillon de tissu colorectal et/ou de cellules colorectales, l'échantillon de tissu colorectal et/ou de cellules colorectales est déterminé comme étant un échantillon sain de tissu colorectal et/ou de cellules colorectales ; et
si le gène soumis à empreinte Z19, Z20, Z21, Z22, Z26 ou Z27 est exprimé avec une perte d'empreinte et/ou une variation du nombre de copies dans les noyaux cellulaires dans l'échantillon de tissu colorectal et/ou de cellules colorectales, l'échantillon de tissu colorectal et/ou de cellules colorectales est déterminé comme étant un échantillon de tissu et/ou de cellules de cancer colorectal ;
ou
(r) la soumission d'un échantillon de tissu et/ou de cellules de peau à une analyse de l'expression des gènes soumis à empreinte Z18, Z19, Z21, Z22, Z25 et Z28 de telle sorte que :
si les gènes soumis à empreinte Z18, Z19, Z21, Z22, Z25 et Z28 ne sont pas exprimés ou sont exprimés normalement dans les noyaux cellulaires dans l'échantillon de tissu et/ou de cellules de peau, l'échantillon de tissu et/ou de cellules de peau est déterminé comme étant un échantillon sain de tissu et/ou de cellules de peau ; et
si le gène soumis à empreinte Z18, Z19, Z21, Z22, Z25 ou Z28 est exprimé avec une perte d'empreinte et/ou une variation du nombre de copies dans les noyaux cellulaires dans l'échantillon de tissu et/ou de cellules de peau, l'échantillon de tissu et/ou de cellules de peau est déterminé comme étant un échantillon de tissu et/ou de cellules de cancer de la peau ;
ou
(s) la soumission d'un échantillon de tissu et/ou de cellules de poumon à une analyse de l'expression des gènes soumis à empreinte Z18, Z19, Z21, Z22, Z25, Z26, Z27 et Z28 de telle sorte que :
si les gènes soumis à empreinte Z18, Z19, Z21, Z22, Z25, Z26, Z27 et Z28 ne sont pas exprimés ou sont exprimés normalement dans les noyaux cellulaires dans l'échantillon de tissu et/ou de cellules de poumon, l'échantillon de tissu et/ou de cellules de poumon est déterminé comme étant un échantillon sain de tissu et/ou de cellules de poumon ; et
si le gène soumis à empreinte Z18, Z19, Z21, Z22, Z25, Z26, Z27 ou Z28 est exprimé avec une perte d'empreinte et/ou une variation du nombre de copies dans les noyaux cellulaires dans l'échantillon de tissu et/ou de cellules de poumon, l'échantillon de tissu et/ou de cellules de poumon est déterminé comme étant un échantillon de tissu et/ou de cellules de cancer du poumon.
